# EUROPEAN PATENT APPLICATION

(11) **EP 4 335 442 A1**
(43) Date of publication of application: **13.03.2024**
(21) Application number: 22867637.5
(22) Date of filing: 02.09.2022
(51) Int. Cl.: A61K 31/7076, A61K 45/06, A61P 35/00, A61P 35/04, A61K 41/00

(54) **USE OF ADENOSINE DIPHOSPHATE RIBOSE AS ADJUVANT THERAPY FOR RADIATION AND/OR ANTICANCER THERAPY**

(30) Priority: 07.09.2021 KR 20210118942
(71) Applicant: Pearlsinmires Co., Ltd., Seoul 07292 (KR)
(72) Inventor: JEONG, Keun-Yeong, Seoul 03690 (KR); PARK, Min Hee, Siheung-si, Gyeonggi-do 14914 (KR)
(74) Representative: HGF
(86) International application number: PCT/KR2022/013186
(87) International publication number: WO 2023/038368

(57) **Abstract**

The present disclosure relates to an anti-cancer therapy using adenosine diphosphate ribose (ADP-Ribose), and specifically, to a single anti-cancer therapy of ADP-ribose and a combination therapy of ADP-ribose with an anti-cancer drug or radiation, and the like.

## Description

### [Technical Field]

The present disclosure relates to an anti-cancer therapy using adenosine diphosphate ribose (ADP-Ribose), and specifically, to a single anti-cancer therapy of ADP-ribose and a combination therapy of ADP-ribose with an anti-cancer drug or radiation, and the like.

### [Background Art]

Radiation therapy is a cancer treatment that uses powerful energy beams such as X-rays to kill cancer cells. During radiation therapy, a high-energy beam emerges from the device, and cancer is treated by aiming the beam toward a precise point in the body. Radiation therapy damages cells by destroying genetic materials (such as DNA, and the like) that control cell growth and division. The ultimate goal of radiation therapy is to target and destroy cancer tissue, avoiding normal and healthy tissue as possible. With advances in computer technology, computed tomography (CT), positron emission tomography (PET), and magnetic resonance imaging (MRI) scans have been developed to accurately compute target tumors to reduce damage to normal tissue. Intensity-controlled radiation therapy and proton beam therapy are performed by aiming beams of photons and protons in multiple directions to a target with little error in order to efficiently remove tumors and to minimize damage to normal organs. Through these methods, side effects caused by radiation therapy are on the decline. However, despite the improvement in treatment technology, many patients have a problem in that cancer easily recurs after treatment due to the inherent resistance of cancer cells to radiation.

Chemotherapy refers to any anti-cancer treatment that uses drugs to kill cancer cells. Chemotherapy may be comprised of a single drug or a combination of drugs, and may be administered intravenously, intramuscularly, subcutaneously, or may be injected into a body cavity, or delivered orally in pill form. Chemotherapy is different from surgery or radiation therapy in that cancer-fighting drugs spread through the blood and circulate all parts of the body to kill or eliminate cancerous (metastatic) cells distant from primary cancer. More than half of all people diagnosed with cancer receive chemotherapy. However, one of the obstacles to treatment through cancer chemotherapy is concern about the side effects of the drugs. Chemotherapy may affect all cells in the body that grow and divide rapidly, including new blood cells in the bone marrow or normal cells such as cells in the mouth, stomach, skin, hair, and reproductive organs. Side effects occur when chemotherapy damages normal cells. Whether or not side effects occur and the severity thereof may vary depending on the type of drug and the response from the first treatment cycle to the next treatment cycle, but is particularly closely related to the dose of the drug. If side effects become severe, treatment should be stopped immediately, and side effects tend to improve gradually after normal and healthy cells are recovered. Sometimes chemotherapy may cause long-term side effects that do not disappear, wherein the side effects may include damage to the heart, lungs, nerves, kidneys, or reproductive organs.

Patients with advanced cancer may experience failure of initial treatment due to unexpected resistance during the treatment process that patients must undergo, and even if treatment such as radiation or chemotherapy is able to be continuously performed without resistance to treatment, the burden of various large and small side effects that must be endured is quite large. In the case of radiation therapy, as a method for continuously maintaining the anti-cancer effect induced by treatment, there have been attempts to control various molecular biological and genetic factors used for suppression of cancer-specific genes induced in a low oxygen environment or DNA repair. However, since known therapies for enhancing radiation therapy effect or overcoming resistance may have, when combined with radiation therapy, the possibility of exacerbating side effects basically caused by radiation therapy such as inflammation, gastrointestinal disorder, nausea, vomiting, and diarrhea, and the like, there are limitations to the active use of the known therapies, and it has not been clearly recognized as a basic therapy for overcoming resistance or enhancing sensitivity to radiation therapy. In the case of chemotherapy, the most obvious way to reduce the side effects that must be endured compared to the efficacy of treatment is to reduce the dose. However, due to the nature of the therapy, the method for reducing the dose itself due to side effects can be regarded as a failure of treatment. Therefore, the results through the methods known to date have been unsatisfactory, and the part to overcome this is sufficient for unmet needs from a medical point of view and remains a very difficult task to solve.

Adenosine diphosphate ribose or ADP-ribose is a biomaterial derived from NAD (nicotinamide adenine dinucleotide) and is known to be involved in post-translational modification as a unit molecule of ADP-ribosylation in cells. The use of exogenous NAD against malignant tumors or infectious diseases is partially disclosed in WO 99/12951, and the use of exogenous ADP-ribose against adenovirus-related diseases or conditions or mucositis is also partially disclosed in WO 2017/143113 or WO 03/099297. However, these documents above do not disclose specific anti-cancer uses or anti-cancer adjuvant uses of ADP-ribose.

### [Disclosure]

### [Technical Problem]

An object of the present disclosure is to improve the efficacy of anti-cancer therapy by utilizing adenosine diphosphate ribose (ADP-ribose).

### [Technical Solution]

In order to achieve the above object, the present inventors have studied and made efforts, and as a result, confirmed that ADP-ribose accumulation in cancer cells could induce disturbance of biochemical action that causes the loss of ADP-ribose homeostasis in cancer cells to exert an anti-cancer effect and significantly improve sensitivity to radiation therapy and/or chemotherapy, and completed the present disclosure. Therefore, the present disclosure provides a method for using ADP-ribose for anti-cancer treatment, a method for combining ADP-ribose with radiation therapy and/or anti-cancer therapy for anti-cancer treatment, an ADP-ribose preparation for anti-cancer treatment, and the like.

### 1. Use of ADP-ribose for anti-cancer treatment

The present inventors completed the present disclosure by specifically confirming the anti-cancer effect of ADP-ribose in various solid carcinomas. Therefore, the present disclosure provides an anti-cancer composition comprising ADP-ribose or a pharmaceutically acceptable salt thereof; a method for treating cancer comprising administering to a patient a therapeutically effective amount of ADP-ribose or a pharmaceutically acceptable salt thereof; use of ADP-ribose or a pharmaceutically acceptable salt thereof for the prevention or treatment of cancer; or use of ADP-ribose or a pharmaceutically acceptable salt thereof for the production of an anti-cancer drug.

### ADP-ribose or pharmaceutically acceptable salt thereof

In the present disclosure, ADP-ribose (adenosine diphosphate ribose) is a compound represented by the following Chemical Formula 1:

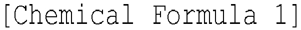

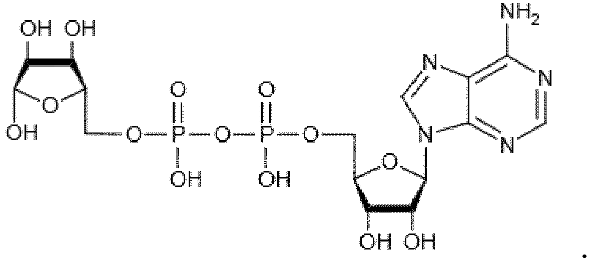

A method for preparing ADP-ribose is known in the art to which the present disclosure pertains. In an embodiment, ADP-ribose is capable of being synthesized via hydrolysis of nicotinamide adenine dinucleotide (NAD+) in the presence of an alkaline base. In addition, ADP-ribose may be isolated in the form of a monovalent or divalent salt of the metal ion of the corresponding base. Alternatively, ADP-ribose is commercially available as a purified raw material (CAS Number: 68414-18-6) .

In the present disclosure, the pharmaceutically acceptable salt refers to salts commonly used in the pharmaceutical industry, and may include, for example, salts of inorganic ions including sodium, potassium, calcium, magnesium, lithium, copper, manganese, zinc, iron, and the like, and salts of inorganic acids such as hydrochloric acid, phosphoric acid, and sulfuric acid, and in addition thereto, may include salts of organic acids such as ascorbic acid, citric acid, tartaric acid, lactic acid, maleic acid, malonic acid, fumaric acid, glycolic acid, succinic acid, propionic acid, acetic acid, orotate acid, acetylsalicylic acid, and the like, and amino acid salts such as lysine, arginine, guanidine, and the like. Further, organic ion salts such as tetramethyl ammonium, tetraethyl ammonium, tetrapropyl ammonium, tetrabutyl ammonium, benzyl trimethyl ammonium, and benzethonium capable of being used in pharmaceutical reactions, purification, and separation processes, may be included. However, the types of salts meant in the present disclosure are not limited by these listed salts. Preferably, the pharmaceutically acceptable salt of ADP-ribose in the present disclosure is not a lithium salt (including mono- and di-lithium salts) of ADP-ribose.

In the present disclosure, ADP-ribose may be used in the form of a prodrug of ADP-ribose such as poly-ADP-ribose (Poly-ADPR) . For example, the ADP-ribose prodrug may be formed by condensing one or more hydroxyl groups of a terminal ribose moiety in ADP-ribose represented by Chemical Formula 1 with a carboxylic acid, an amino acid, a fatty acid, or a combination thereof.

### Anti-cancer effect of ADP-ribose

The present inventors found that the anti-cancer composition of the present disclosure could accumulate ADP-ribose in cancer cells and induce disturbance of biochemical action in cancer cells, thereby exerting an anti-cancer effect.

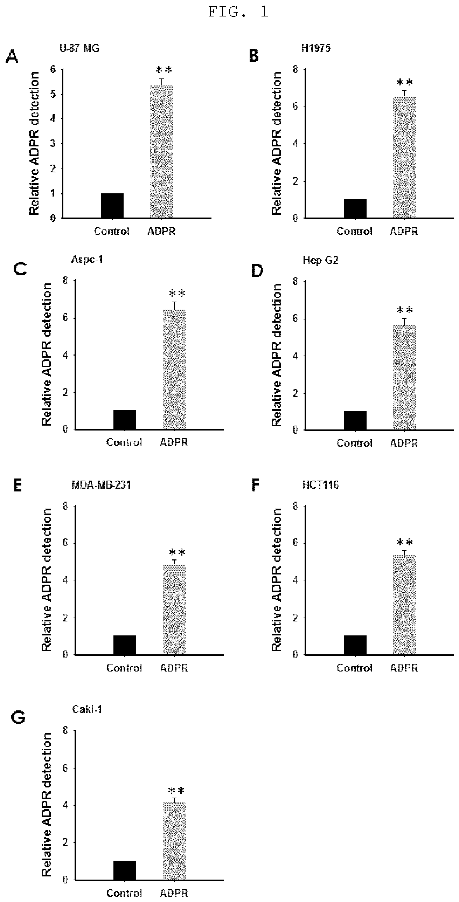

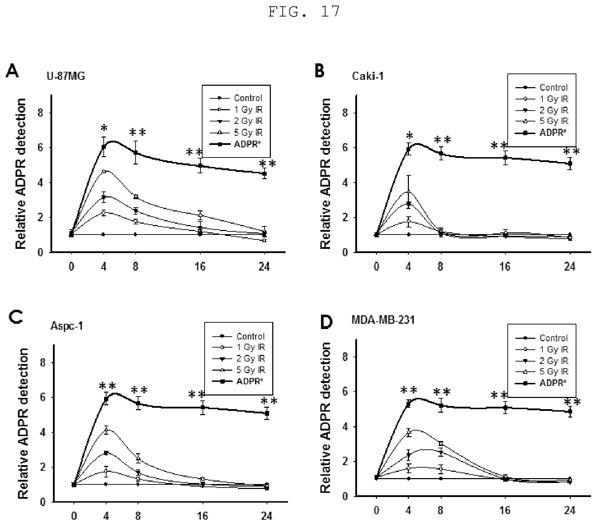

Specifically, the present inventors confirmed that when cell lines of various solid cancers (brain cancer, lung cancer, pancreatic cancer, liver cancer, breast cancer, colon cancer, and kidney cancer) were treated with ADP-ribose (concentration of several to several tens of µM), the concentration of ADP-ribose in cancer cells significantly increased (Example 1; FIG. 1). In particular, the concentration of ADP-ribose in cancer cells increased only temporarily (several hours) upon irradiation which is used as one of the anti-cancer therapies, but it was found that at the time of external ADP-ribose treatment, the concentration of ADP-ribose in cancer cells significantly and continuously increased compared to that upon irradiation (Example 7; FIG. 17).

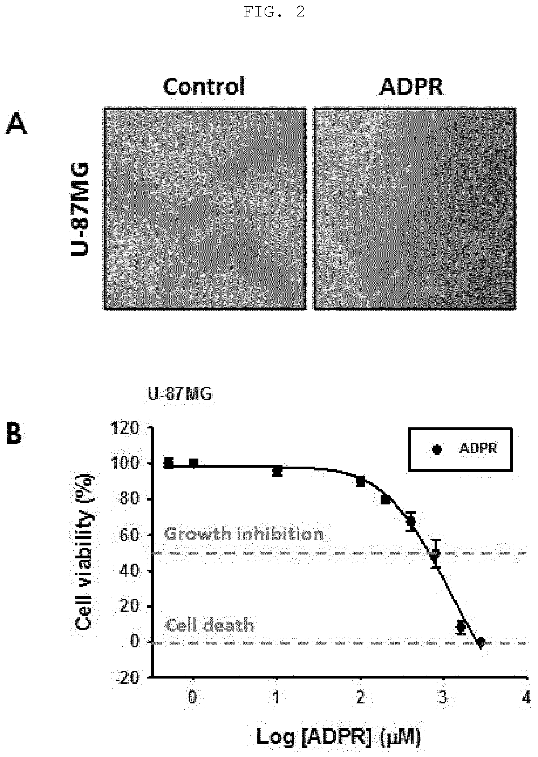

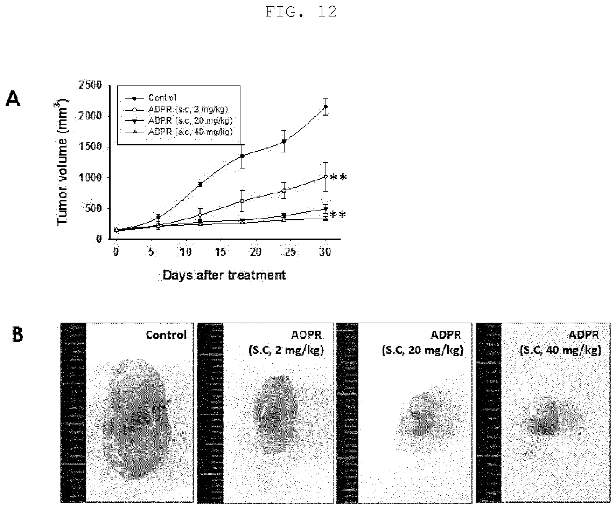

The present inventors expected that when ADP-ribose is artificially accumulated inside cancer cells, an effective anti-cancer effect could be exerted by a breakdown of the biochemical homeostasis of ADP-ribose maintained in cancer cells. The present inventors attempted to concretely confirm this prediction, and as a result, found that ADP-ribose at a low-concentration (concentration of several to several tens of µM) exhibited remarkable anti-cancer effects on various solid cancers (brain cancer, lung cancer, pancreatic cancer, liver cancer, breast cancer, colon cancer, kidney cancer, gastric cancer, and ovarian cancer) (Example 2; FIGS. 2 to 9, Example 4; FIGS. 12 to 14).

It was confirmed from these findings that ADP-ribose or a pharmaceutically acceptable salt thereof could be usefully employed for cancer treatment.

In the present disclosure, cancer, also called malignant tumor or neoplasia, is a disease related to the control of cell death, and refers to a condition or disease caused by excessive proliferation of cells as a result of the loss of normal cell death balance. In some cases, these abnormal hyperproliferative cells invade surrounding tissues and organs to form masses and destroy or transform normal structures in the body.

Cancer capable of being alleviated, reduced or treated by the anti-cancer composition of the present disclosure is not particularly limited as long as proliferation, invasion, and metastasis are able to be inhibited by disturbance of the biochemical action process, but is preferably solid cancer in which proliferation, invasion, metastasis, and the like, are able to be inhibited by disturbance of the ADP-ribose biochemical action in cells. Solid cancer may be breast cancer, cervical cancer, glioma, brain cancer, melanoma, head and neck cancer, lung cancer, bladder cancer, prostate cancer, leukemia, kidney cancer, liver cancer, colon cancer, rectal cancer, colon cancer, pancreatic cancer, gastric cancer, gallbladder cancer, ovarian cancer, lymphoma, osteosarcoma, cervical cancer, oral cancer, bronchial cancer, nasopharyngeal cancer, laryngeal cancer, skin cancer, squamous cell carcinoma, thyroid cancer, parathyroid cancer or ureteral cancer, but is not limited thereto. The cancer may be a cancer in which a specific gene is mutated. Preferably, solid cancer is at least one selected from the group consisting of brain cancer, lung cancer, pancreatic cancer, liver cancer, breast cancer, colon cancer, kidney cancer, gastric cancer, and ovarian cancer.

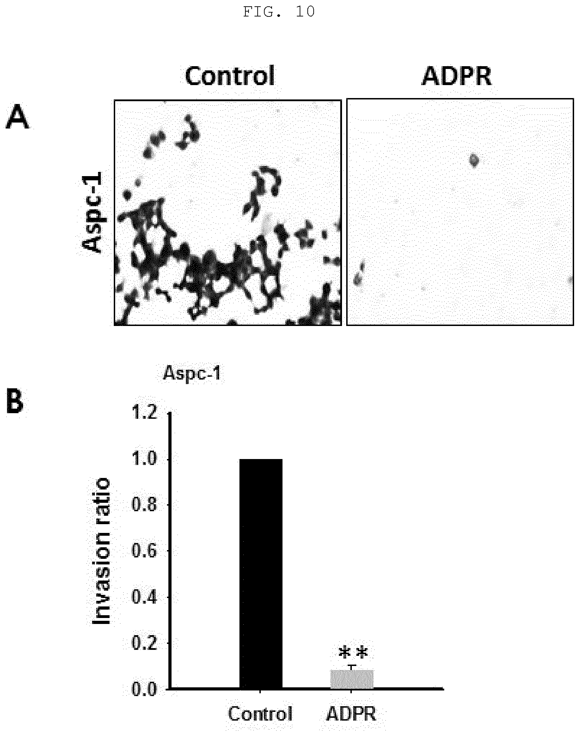

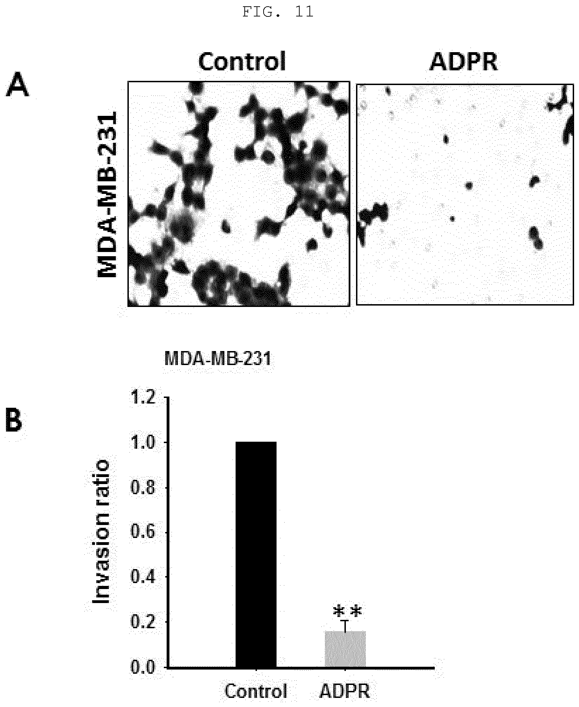

In the present disclosure, cancer includes primary cancer and metastatic cancer. The anti-cancer composition of the present disclosure is effective in inhibiting the ability of cancer cells to invade and metastasize. Specifically, the present inventors confirmed that ADP-ribose at a low-concentration (concentration of several to several tens of µM) exhibited significant anti-cancer effects against metastatic cancers (pancreatic cancer and breast cancer) (Example 3; FIGS. 10 and 11). Thus, the composition of the present disclosure is able to be usefully employed for the treatment of solid cancers, particularly metastatic solid cancers (for example, metastatic pancreatic cancer, and metastatic breast cancer).

### Administration route, regimen and dose

The anti-cancer composition according to the present disclosure may be administered through any common route of administration as long as the anti-cancer composition is able to reach the target tissue. For example, the anti-cancer composition may be administered through a route such as intraperitoneal administration, intravenous administration, intramuscular administration, subcutaneous administration, intradermal administration, transdermal patch administration, oral administration, intranasal administration, intrapulmonary administration, intrarectal administration, or the like, depending on the purpose. When administered orally, the anti-cancer composition may be administered into the oral cavity in an unformulated form or in a formulated form to coat the active ingredient or to protect it from degradation in the stomach or an oral patch form. Further, the anti-cancer composition of the present disclosure may be administered by any device capable of delivering an active ingredient to a target cancer cell.

Specifically, the present inventors confirmed through animal experiments using mice as subjects that when ADP-ribose was administered subcutaneously, intravenously, or orally, anti-cancer effects against various solid cancers (pancreatic cancer, kidney cancer, and pancreatic cancer) were exhibited (Example 4; FIGS. 12 to 14).

The anti-cancer composition according to the present disclosure may be administered in a therapeutically effective amount, which means an amount sufficient to prevent or treat a disease with a reasonable benefit/risk ratio applicable to medical treatment or prevention. The therapeutically effective amount level may be determined depending on factors including the severity of the disease, the activity of the drug, the patient's age, weight, health, sex, the patient's sensitivity to the drug, the administration time, the route of administration, and excretion rate, of the composition of the present disclosure used, the treatment period, drugs used in combination or concurrently with the composition of the present disclosure used, and other factors well known in the medical field. The anti-cancer composition of the present disclosure may be administered once or may be divided and administered several times a day.

The anti-cancer composition of the present disclosure may be administered alone or in combination with one or more anti-cancer therapies. When administered in combination, one or more anti-cancer therapies include chemotherapy, immuno-anti-cancer therapy, radiotherapy, surgery, nanomedicine, or a combination thereof. The type and number of the anti-cancer therapy are not particularly limited as long as the anti-cancer therapy is capable of enhancing the anti-cancer effect of ADP-ribose. For example, the anti-cancer composition of the present disclosure may be used for adjuvant therapy before and after surgery, and may be used simultaneously with or before and after radiation therapy and/or anti-cancer therapy as described below.

### 2. Combination of radiation therapy with ADP-ribose for anti-cancer treatment

The present inventors found that the effect of anti-cancer treatment significantly increased when radiation therapy and ADP-ribose were used in combination. Therefore, the present disclosure provides an anti-cancer composition comprising ADP-ribose or a pharmaceutically acceptable salt thereof used in combination with radiation therapy; a cancer treatment method comprising administering a therapeutically effective amount of ADP-ribose or a pharmaceutically acceptable salt thereof to a patient in combination with radiation therapy; an anti-cancer adjuvant for radiation therapy comprising ADP-ribose or a pharmaceutically acceptable salt thereof; a radiation sensitizer comprising ADP-ribose or a pharmaceutically acceptable salt thereof; or use of the ADP-ribose or a pharmaceutically acceptable salt thereof as an adjuvant for radiation therapy.

In the present disclosure, the anti-cancer adjuvant for radiation therapy refers to an agent capable of improving, enhancing or increasing the anti-cancer effect of radiation therapy when administered before or after radiation therapy.

### Radiation therapy

As used herein, the term "radiation therapy" refers to a treatment activity of irradiating cancer cells or tumor tissue with radiation for the purpose of killing cancer cells. The anti-cancer radiation therapy is a standard treatment for controlling unresectable or inoperable tumors, or tumor metastasis, and is based on the principle that radiation delivered to a target site causes the death of reproductive cells. The anti-cancer radiation therapy in the present disclosure may be ionizing radiation therapy, electromagnetic radiation therapy, brachytherapy, or external beam radiation therapy, but is not limited thereto.

Since the composition containing ADP-ribose or a pharmaceutically acceptable salt thereof according to the present disclosure exhibits a synergistic anti-cancer effect when used in combination with radiation therapy, the composition may be usefully employed as an anti-cancer adjuvant for radiation therapy or as a radiation sensitizer to improve radiation sensitivity, wherein the type of applicable cancer is not particularly limited. As an example, the cancer may be solid cancer such as brain cancer, lung cancer, pancreatic cancer, liver cancer, breast cancer, colon cancer, kidney cancer, gastric cancer, or ovarian cancer, but is not limited thereto.

As an embodiment, the solid cancer may be resistant to radiation therapy. Since the anti-cancer effect of radiation therapy is exhibited by the formation of DNA fragments, resistance to radiation therapy is generally determined by a mechanism capable of repairing DNA damage induced by radiation therapy. As a method for increasing the anti-cancer efficacy of radiation therapy, a number of attempts have been made to inhibit DNA repair mechanisms, and the sensitivity of radiation therapy may be increased when the repair of broken DNA strands is blocked. The two main types of DNA damage are single-strand breaks (SSB) and double-strand breaks (DSB). Therefore, the categories of two repair pathways targeting SSB and DSB may be described as examples. Base Excision Repair (BER) is one of several pathways involved in the repair of selected types of DNA SSBs.

PARP1 plays an important role in the BER of DNA SSBs through a process known as ADP-ribosylation. In the nucleus, PARP1 detects damage to SSB DNA, and for damage repair, recruits DNA repair complexes via ADP-ribosylation to SSB sites. Since over-accumulation of poly-ADP-ribose synthesized through ADP-ribosylation by PARP-1 activity ultimately leads to cell death, in order to prevent this phenomenon, cancer cells activate survival signaling activities by activating the degradation of poly-ADP-ribose through proteasomes such as PARG, ARH3, and the like. The present inventors noticed that the accumulation of ADP-ribose or ADP-ribose polymer in cancer cells could act as a vehicle capable of disturbing these biochemical survival mechanisms of cancer cells, thereby making it possible to be used as an important anti-cancer adjuvant to overcome resistance to radiation therapy.

In a specific embodiment of the present disclosure, it was confirmed that when the ADP-ribose or a pharmaceutically acceptable salt thereof of the present disclosure was treated in combination with low-dose radiation in different cancer types, the responsiveness to radiation therapy was enhanced in all cancer types. Therefore, ADP-ribose or a pharmaceutically acceptable salt thereof of the present disclosure is able to enhance the response to radiation therapy to thereby be very useful as an anti-cancer adjuvant, and even when irradiated with radiation at a tolerable dose, excellent anti-cancer activity may be obtained, which may minimize side effects that may occur due to radiation.

Specifically, the present inventors confirmed that the ADP-ribose treatment in various solid cancers could maintain an increase in intracellular ADP-ribose concentration higher than the concentration of ADP-ribose in cancer cells that elevated upon irradiation (Example 7; FIG. 17). The anti-cancer effect of irradiation may be expected to have an anti-cancer effect through the cell death action by damaging genetic material, but to overcome the anti-cancer effect, cancer cells continually repair DNA strands through ADP-ribosylation. However, as can be appreciated in FIG. 17, if ADP-ribose, which increases only temporarily for the repair of DNA strands destroyed by irradiation, is supported to accumulate continuously, biochemical disturbance in the DNA repair process of cancer cells may be caused, which may be expected to have a synergistic anti-cancer effect of radiation therapy, and furthermore, a possibility of using anti-cancer radiation at a reduced dose to overcome resistance to radiation therapy and reduce side effects.

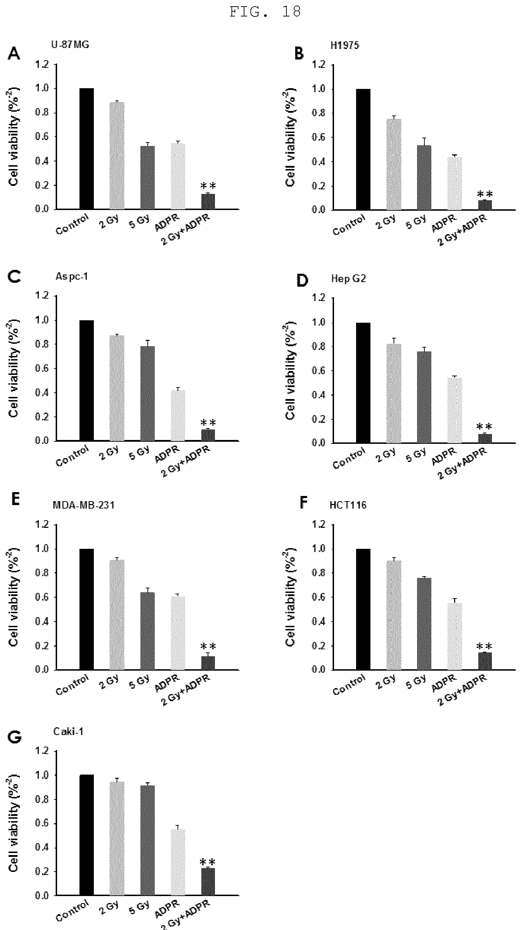

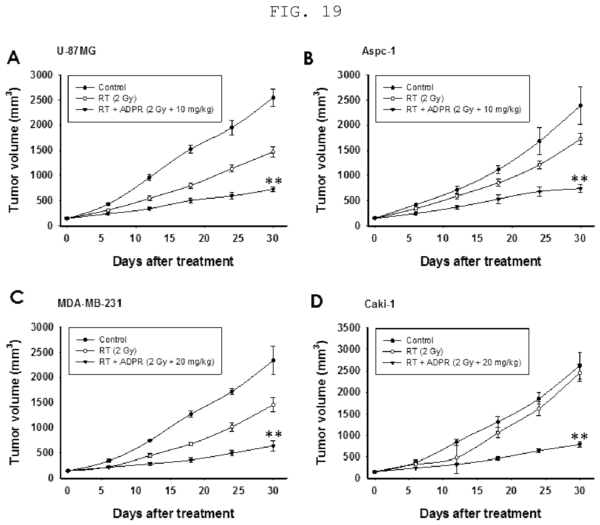

In addition, the present inventors confirmed that the combination treatment of ADP-ribose administration and radiation therapy showed a synergistic anti-cancer effect against various solid cancers (brain cancer, lung cancer, pancreatic cancer, breast cancer, colon cancer, and kidney cancer) (Example 8; FIG. 18, and Example 9; FIG. 19).

### Administration route, regimen and dose

Irradiation therapy is generally defined in terms of radiation absorbed dose (Gray; Gy), time, and classification, and must be carefully defined by the oncologist. The radiation dose that is acceptable to the patient depends on a number of considerations, but the two most important factors are the location of the tumor relative to other vital structures or organs in the body and the extent to which the tumor has spread. A typical course of treatment for a patient receiving radiation therapy may be, for example, a treatment schedule of a dose of about 1.8 to 2.0 Gy once a day for 5 days a week, i.e., over a period of 1 to 6 weeks with a total dose administered to the patient of 10 to 80 Gy, or may be a treatment schedule with multi-fractionated radiation with low-doses, such as several dozens of 1-10 cGy or 5-40 cGy dose, but is not limited thereto.

The present inventors confirmed in a tumor mouse model that a combination treatment of 2 Gy dose radiation therapy and ADP-ribose administration showed a synergistic anti-cancer effect against various solid cancers (Example 8; FIG. 18, and Example 9; FIG. 19). However, the radiation absorbed dose of radiation therapy capable of being used in combination with the anti-cancer composition of the present disclosure is not limited thereto, and a single dose may be 1 to 10 Gy, and a total dose may be about 1 to 100 Gy, but is not limited thereto. For example, the total radiation dose irradiated to the patient may be about 1 cGy to about 100 Gy or about 1 cGy to about 50 Gy. In some embodiments, the subject is and/or may be receiving radiation therapy. The radiation therapy may include one or more radiation therapies in which radiation is administered 5 days per week for 1 to 10 weeks. As may be appreciated by those skilled in the art, radiation therapy may be performed over a specific period of time (for example, 1-10 weeks) and may not be administered to the subject continuously, but rather intermittently.

The route of administration, regimen, and dose of ADP-ribose used in combination with radiation therapy is the same as described above, and ADP-ribose may be administered before or after direct radiation therapy. In addition, as described below, chemotherapy may be used in combination.

### 3. Combination of chemotherapy with ADP-ribose for anti-cancer treatment

The present inventors found that the effect of anti-cancer treatment was significantly increased when chemotherapy and ADP-ribose were used in combination. Therefore, the present disclosure provides an anti-cancer adjuvant or sensitizer in chemotherapy comprising ADP-ribose or a pharmaceutically acceptable salt thereof; an anti-cancer composition or combination comprising (i) ADP-ribose or a pharmaceutically acceptable salt thereof and (ii) a second anti-cancer drug; a cancer treatment method comprising administering a therapeutically effective amount of ADP-ribose or a pharmaceutically acceptable salt thereof and a second anti-cancer drug in combination; and use of ADP-ribose or a pharmaceutically acceptable salt thereof as an anti-cancer adjuvant or sensitizer in chemotherapy for the prevention or treatment of cancer.

In the present disclosure, the anti-cancer adjuvant for chemotherapy is an agent capable of improving, enhancing or increasing the anti-cancer effect of the anti-cancer drug, and means an agent capable of improving, enhancing or increasing the anti-cancer effect of the anti-cancer drug when used together with the anti-cancer drug.

### Anti-cancer drugs able to be used in combination with ADP-ribose

In the present disclosure, chemotherapy is a cancer treatment method using one or more chemotherapeutic agents as anti-cancer drugs. In the present disclosure, as the second anti-cancer drug able to be used in combination with ADP-ribose or a pharmaceutically acceptable salt thereof, an appropriate type thereof may be selected depending on the type and progress of cancer to cure, control, and alleviate symptoms of cancer. For example, the second anti-cancer drug may be at least one selected from a cytotoxic anti-cancer drug, a targeted anti-cancer drug, an immuno-oncology drug, a metabolic anti-cancer drug or a combination thereof. In the present disclosure, the cytotoxic anti-cancer drug is a drug that exhibits an anti-cancer effect by attacking cancer cells that divide indiscriminately at a faster rate than normal cells, which is the same as that commonly used in the art to which the present disclosure belongs. The cytotoxic anti-cancer drug includes alkylating agents, antimetabolites, and natural anti-cancer drugs.

The alkylating agent may be nitrogen mustards (for example, cyclophosphamide, chlormethine, uramustine, melphalan, chlorambucil, ifosfamide, bendamustine, and the like), alkyl sulfonates (for example, busulfan, procarbazine, and the like), nitrosoureas (for example, carmustine, lomustine, streptozocin, and the like), platinum-based alkylating agents (for example, cisplatin, carboplatin, dicycloplatin, eptaplatin, lobaplatin, myriplatin, nedaplatin, oxaliplatin, picoplatin, satraplatin, triplatin tetranitrate, and the like), but is not limited thereto. The alkylating agent may cause the destruction of cancer cells by binding to DNA in cancer cells and damaging the DNA structure.

The antimetabolite may be pyrimidine derivatives (for example, 5-fluorouracil, capecitabine, cytarabine, gemcitabine, fludarabine, and the like), folate derivatives (for example, methotrexate, pemetrexed, and the like), purine derivatives (for example, mercaptopurine, and the like), but is not limited thereto. The antimetabolite may induce cancer cell death by inhibiting the metabolism necessary for cell survival and DNA replication.

The natural anti-cancer drug may include topoisomerase inhibitors (camptothecin, epipodophyllotoxin, taxane-based drugs), antibiotics (for example, dactinomycin, doxorubicin, daunorubicin, mitomycin, phleomycin, idarubicin, mitoxantrone HCl, and the like), but is not limited thereto.

In the present disclosure, the targeted anti-cancer drug is an anti-cancer drug that induces the death of cancer cells by inhibiting a target protein (receptor or enzyme) involved in cancer growth, which is the same as that commonly used in the art to which the present disclosure belongs. The targeted anti-cancer drug includes target protein (tyrosine kinase, and the like) inhibitory small molecule compounds and monoclonal antibodies.

In the present disclosure, the targeted anti-cancer drug may be a receptor tyrosine kinase inhibitor targeting at least one target selected from the group consisting of VEGF/VEGFR, EGFR, and HER2.

In an embodiment, the targeted anti-cancer drug capable of being administered in combination with ADP-ribose is a VEGF/VEGFR inhibitor. In the present disclosure, examples of the VEGF/VEGFR inhibitor may include monoclonal antibodies such as bevacizumab, ramucirumab, and ranibizumab, together with small molecule compounds such as axitinib, cabozantinib, lapatinib, lenvatinib, pazopanib, regorafenib, sorafenib, sunitinib, and vandetanib, but the VEGF/VEGFR inhibitor is not limited thereto. In the present disclosure, the cancer capable of being treated by the combination administration of ADP-ribose and the VEGF/VEGFR inhibitor is solid cancer, preferably brain cancer or liver cancer.

In an embodiment, the targeted anti-cancer drug capable of being administered in combination with ADP-ribose is an EGFR inhibitor. In the present disclosure, examples of the EGFR inhibitor may include monoclonal antibodies such as cetuximab, panitumumab, zalutumumab, nimotuzumab, and matuzumab together with small molecule compounds such as osimertinib, gefitinib, erlotinib, afatinib, brigatinib, icotinib, and vandetanib, but the EGFR inhibitor is not limited thereto. The cancer capable of being treated by the combination administration of ADP-ribose and the EGFR inhibitor in the present disclosure is solid cancer, preferably lung cancer.

In an embodiment, the targeted anti-cancer drug capable of being administered in combination with ADP-ribose is a HER2 inhibitor. In the present disclosure, examples of the HER2 inhibitor may include monoclonal antibodies such as trastuzumab, pertuzumab, and margetuximab together with small molecule compounds such as lapatinib, neratinib, and afatinib, but the HER2 inhibitor is not limited thereto. The cancer capable of being treated by the combination administration of ADP-ribose and the HER2 inhibitor in the present disclosure is solid cancer, preferably breast cancer.

In addition, examples of the targeted anti-cancer drug of the present disclosure may also include Bcr-Abl targeted anti-cancer drugs such as imatinib, dasatinib, and nilotinib; Src targeted anti-cancer drugs such as bosutinib; JAK targeted anti-cancer drugs such as lestaurtinib, ruxolitinib, and pacritinib; MAP2 Kinase targeted anti-cancer drugs such as cobimetinib, selumetinib, trametinib, and binimetinib; MEL4-ALK targeted anti-cancer drugs such as ceritibin and crizotinib, and the like.

The second anti-cancer drug of the present disclosure may be a combination of one or more cytotoxic anti-cancer drugs and/or targeted anti-cancer drugs, which may be administered at the same time or at different times. For example, the second anti-cancer drug may be at least one selected from the group consisting of nitrogen mustard, imatinib, oxaliplatin, rituximab, erlotinib, trastuzumab, gefitinib, bortezomib, sunitinib, carboplatin, sorafenib, bevacizumab, cisplatin, cetuximab,viscumalbum, asparaginase, tretinoin, hydroxycarbamide, dasatinib, estramustine, gemtuzumab ozogamicin, ibritumomabtusetan, heptaplatin, methylaminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pirarubicin, aclarubicin, pepromycin, temozolomide, busulfan, ifosfamide, cyclophosphamide, melpharan, altretamine, dacarbazine, thiotepa, nimustine, chlorambucil, mitolactol, lomustine and carmustine, but is not limited thereto.

In the present disclosure, cancer immunotherapy is a cancer treatment method that activates the body's immune system through an immuno-oncology drug to fight cancer cells. In the present disclosure, the immuno-oncology drug may include immune checkpoint inhibitors, immune cell therapeutic agents, anti-cancer vaccines, and antibody-drug conjugates, wherein the appropriate type thereof may be selected to cure, control, and alleviate symptoms of cancer depending on the type and stage of cancer.

In an embodiment, the immuno-oncology drug may be an immune checkpoint inhibitor, and may be one or more selected from the group consisting of a PD-1 antibody, a PD-L1 antibody, a CTLA-4 antibody, a CD28 antibody, a KIR antibody, a TCR antibody, a LAG-3 antibody, a TIM-3 antibody, a TIGIT antibody, an A2aR antibody, an ICOS antibody, an OX40 antibody, a 4-1BB antibody, and a GITR antibody. For example, the immune checkpoint inhibitor may be PD-1 antibodies such as nivolumab, pembrolizumab, cemiplimab, pidilizumab, toripalimab, and the like; PD-L1 antibodies such as atezolizumab, avelumab, duralumab, and the like; CTLA-4 antibodies such as ipilimumab and tremelimumab, or all of them.

In an embodiment, the immuno-oncology drug may be an cell therapeutic agent, and may be a chimeric antigen receptor-natural killer (CAR)-NK agent or a chimeric antigen receptor (CAR)-T agents such as tisagenlecleucel or axicabtagene ciloleucel, but is not limited thereto.

In the present disclosure, the metabolic anti-cancer drug refers to a drug that kills cancer cells by being involved in the growth and survival of cancer cells or involved in several essential metabolic reactions, such as supplying nutrients to cancer cells, and the like. The anti-metabolism agent may include, for example, IM-156, 3-bromopyruvic acid (3BP), NYH817100, WZB117, GNE-140, AZ93, AZD3965, CPI-613, MKT-077, CB-839, CB-1158, CPI-444, TVB-2640, NDI-010976, TCD-717, ADI-PEG20, Epacadostat, Indoximod, PX478, CPI-0610, RTA402, APO866, GMX1778, AG-221 or AG-120, but is not limited thereto.

The ADP-ribose of the present disclosure may be administered in combination with the second anti-cancer drug in a form that exists independently of each other, and may be administered in a state in which a physical/chemical bond is formed with the second anti-cancer drug by any known method depending on the purpose. For example, ADP-ribose may be used in a state directly coupled with the second anti-cancer drug, or may be used in a state connected to the second anti-cancer drug through a known linker, and the application method is not particularly limited as long as the ADP-ribose of the present disclosure exhibits a synergistic anti-cancer effect by acting together with the second anti-cancer drug.

### Synergistic anti-cancer effect of ADP-ribose in combination with anti-cancer drug

Since the composition containing ADP-ribose or a pharmaceutically acceptable salt thereof according to the present disclosure exhibits a synergistic anti-cancer effect when used in combination with chemotherapy, the composition may be usefully utilized as an anti-cancer adjuvant for chemotherapy or a sensitizer for anti-cancer therapy. In the anti-cancer adjuvant or the sensitizer for chemotherapy, the cancer may be the above-described solid cancer. The solid cancer may be a solid cancer that is resistant to radiation therapy.

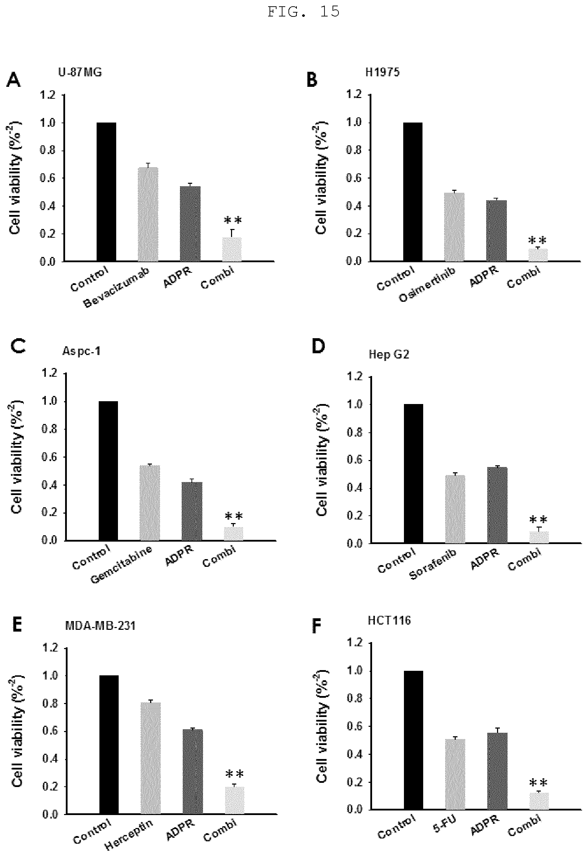

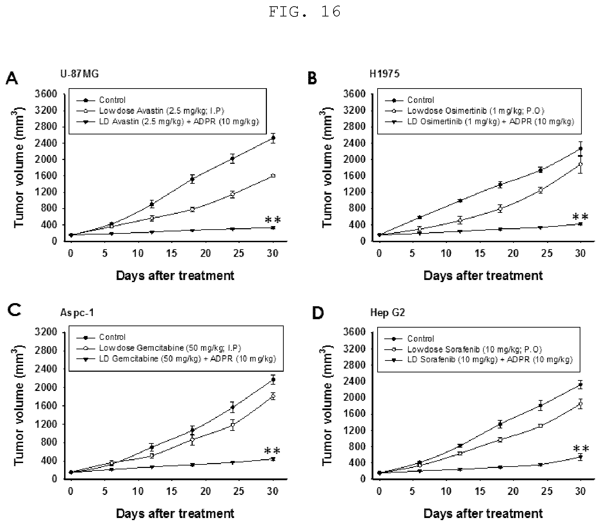

The anti-cancer effect, represented by the reduction of cancer cell survival, and the like, by existing anti-cancer drugs, may be induced by ADP-ribose signaling, and when ADP-ribose is induced to be actively employed in the death process by existing anti-cancer drug, a synergistic anti-cancer effect may be expected even if the existing anti-cancer drug is treated at a concentration below the required concentration. Specifically, the present inventors confirmed that the combination administration of ADP-ribose and a second active agent exhibited a synergistic anti-cancer effect against various solid cancers (brain cancer, lung cancer, pancreatic cancer, liver cancer, breast cancer, and colon cancer) (Example 5; FIG. 15, and Example 6; FIG. 16).

In an embodiment, the cytotoxic anti-cancer drug capable of being administered in combination with ADP-ribose may be an antimetabolite anti-cancer drug, specifically a pyrimidine derivative, and more specifically gemcitabine or 5-fluorouracil. In this case, the cancer capable of being treated according to the combination therapy is solid cancer, specifically pancreatic cancer or colon cancer. The present inventors confirmed the synergistic anti-cancer effect according to the combination administration of ADP-ribose and gemcitabine in pancreatic cancer (Example 5-3; FIG. 15C; and Example 6-3; FIG. 16C), and the synergistic anti-cancer effect according to the combination administration of ADP-ribose and 5-fluorouracil in colon cancer (Example 5-6; FIG. 15F) .

In an embodiment, the targeted anti-cancer drug capable of being administered in combination with ADP-ribose may be a VEGF/VEGFR inhibitor, specifically an anti-VEGF monoclonal antibody. In this case, the cancer capable of being cured may be solid cancer, specifically brain cancer or liver cancer. The present inventors confirmed the synergistic anti-cancer effect according to the combination administration of ADP-ribose and the anti-VEGF monoclonal antibody (bevacizumab) in brain cancer (Example 5-1; FIG. 15A, and Example 6-1; FIG. 16A), and confirmed the synergistic anti-cancer effect according to the combination administration of ADP-ribose and sorafenib in liver cancer (Example 5-4; FIG. 15D, and Example 6-4; FIG. 16D).

In an embodiment, the targeted anti-cancer drug capable of being administered in combination with ADP-ribose may be an EGFR inhibitor, specifically a small molecule EGFR inhibitor. In this case, the cancer capable of being cured may be solid cancer, specifically lung cancer. The present inventors confirmed the synergistic anti-cancer effect according to the combination administration of ADP-ribose and the EGFR inhibitor (osimertinib), in lung cancer (Example 5-2; FIG. 15B, and Example 6-2; FIG. 16B).

In an embodiment, the targeted anti-cancer drug capable of being administered in combination with ADP-ribose may be a HER2 inhibitor, specifically an anti-HER2 monoclonal antibody. In this case, the cancer capable of being cured may be solid cancer, specifically breast cancer. The present inventors confirmed the synergistic anti-cancer effect according to the combination administration of ADP-ribose and the anti-HER2 monoclonal antibody (trastuzumab), in breast cancer (Examples 5-5; FIG. 15E).

### Administration route, regimen and dose

In the anti-cancer composition or combination of the present disclosure, ADP-ribose or a pharmaceutically acceptable salt thereof and the second anti-cancer drug may be administered at the same time or at different times. For example, in the composition or combination, ADP-ribose or a pharmaceutically acceptable salt thereof may be administered before or after the anti-cancer therapy with the second anti-cancer drug. Each regimen may be performed by running one dosing cycle or by repeating the dosing cycle several times, but is not limited thereto.

In an embodiment, the administration schedule of the second anti-cancer drug and ADP-ribose, which confirmed the synergistic anti-cancer effect in a tumor mouse model (FIG. 16), is shown in Table 1 below, and the patient's preferred dose of the anti-cancer composition and the second anti-cancer drug of the present disclosure may be converted by considering the mouse optimal dose specifically identified in the present disclosure, mouse-human dose-response relationship, and no-observed-adverse-effect level (NOAEL), and the like.

**[Table 1]**

| Carcinoma | Second anti-cancer drug | ADP-ribose |
|---|---|---|
| Brain cancer | Bevacizumab 25 mg/kg, intraperitoneal administration twice a week | 10 mg/kg subcutaneous administration 3 times a week |
| Lung cancer | Osimertinib 1 mg/kg, oral administration twice a week | 10 mg/kg subcutaneous administration 3 times a week |
| Pancreatic cancer | Gemcitabine 50 mg/kg, intraperitoneal administration twice a week | 10 mg/kg subcutaneous administration 3 times a week |
| Liver cancer | Sorafenib 10 mg/kg, oral administration 5 times a week | 10 mg/kg subcutaneous administration 3 times a week |

ADP-ribose or a pharmaceutically acceptable salt thereof and the second anti-cancer drug according to the present disclosure may be administered, respectively, in an amount sufficient to treat or prevent disease at a reasonable benefit/risk ratio applicable to medical treatment or prevention. Each effective dose level may be determined depending on factors including the severity of the disease, the activity of the drug, the patient's age, weight, health, sex, the patient's sensitivity to the drug, the administration time, the route of administration, and excretion rate, of the composition of the present disclosure used, the treatment period, drugs used in combination or concurrently with the composition of the present disclosure used, and other factors well known in the medical field.

### 4. ADP-ribose preparation for anti-cancer treatment

The weight percent (wt%) of ADP-ribose or a pharmaceutically acceptable salt thereof contained in the above-described anti-cancer composition is not particularly limited thereto, but may be 0.0001 to 90 wt%, specifically 0.001 to 50 wt%, more specifically 0.01 to 20 wt% based on the total weight of the final composition.

In addition, the anti-cancer composition may be prepared in the form of a pharmaceutical composition or a food composition.

When the anti-cancer composition of the present disclosure is prepared in the form of a pharmaceutical composition, the pharmaceutical composition may be prepared by further containing appropriate carriers, excipients or diluents commonly used in the preparation of pharmaceuticals.

Specifically, the pharmaceutical composition of the present disclosure may be prepared into preparations for various administration routes according to conventional methods, respectively.

In an embodiment, the pharmaceutical composition of the present disclosure may be prepared as a preparation for oral administration. Specifically, the composition may be prepared in tablets (for example, orally disintegrating tablets, chewable tablets, effervescent tablets, dispersing tablets, dissolving tablets), capsules (for example, hard capsules, soft capsules), granules (for example, slow-release granules, enteric granules, effervescent granules), powders, oral liquids (for example, elixirs, suspensions, emulsions, lemonades, aromatic waters, preservatives and precipitates, spirits, tinctures), syrups, oral jellies, medicinal tea, extracts (for example, soft extracts, dry extracts), fluid extracts, or pills.

In an embodiment, the pharmaceutical composition of the present disclosure may be prepared as a preparation for oral administration. Specifically, the composition may be prepared in oral tablets (for example, troches, sublingual tablets, buccal tablets, adhesive tablets, chewing gum), oral liquids (for example, gargles), oral sprays, oral semi-solids (for example, oral creams, oral gels, oral ointments), and oral dissolving films.

In an embodiment, the pharmaceutical composition of the present disclosure may be prepared as an injectable preparation. In this case, the injectable preparation may be prepared in the form of ampoules, vials, prefilled syringes, cartridges, and the like, and more specifically, may be prepared as infusion solutions, freeze-dried injections, powder injections, transplants, long-acting injections, dialysis and perfusion preparations.

In an embodiment, the pharmaceutical composition of the present disclosure may be prepared into a preparation for dialysis and perfusion. Specifically, the composition may be prepared as dialysis agents (for example, peritoneal dialysis agents, hemodialysis agents) or perfusates.

In an embodiment, the pharmaceutical composition of the present disclosure may be prepared into preparation for bronchial or pulmonary administration. Specifically, the composition may be prepared as an inhalant (for example, inhalation powder, inhalation liquid, or inhalation aerosol).

In an embodiment, the pharmaceutical composition of the present disclosure may be prepared into preparation for ocular administration. Specifically, the composition may be prepared as eye drops or ointments.

In an embodiment, the pharmaceutical composition of the present disclosure may be prepared as preparations for nasal administration (for example, nasal powder, nasal solution), enemas, preparations for rectal administration (for example, suppositories, rectal semi-solid preparations, enemas) or vaginal preparations (for example, vaginal tablets, vaginal suppositories), skin application preparations (for example, external solids, external solutions, aerosols, ointment, cream, gel, transdermal absorbent, cataplasma, plaster, paste).

When the pharmaceutical composition is used for oral administration, it may be prepared as a sustained-release preparation through appropriate encapsulation, enteric coating, polymer blend, and the like.

In an embodiment, the sustained-release preparation may be prepared as a long-acting preparation.

In an embodiment, the long-acting preparation may be mixed with a polymer and a lipid in an appropriate ratio.

When the anti-cancer composition of the present disclosure is prepared in the form of a food composition, the food composition may contain additional ingredients that are commonly used in food to improve odor, taste, vision, and the like. For example, food additives may be added. The additive is selected according to the type of food and used in an appropriate amount.

The food composition may be prepared as a functional food, wherein the functional food is the same term as food for special health use (FoSHU), which refers to food with high medicinal and medical effects processed to efficiently show bioregulatory functions in addition to nutritional supply. The health functional food may be prepared in various forms such as tablets, capsules, powders, granules, liquids, and pills in order to obtain useful effects for the improvement of cancer.

### [Advantageous Effects]

The composition containing adenosine diphosphate ribose (ADP-ribose) or a pharmaceutically acceptable salt thereof according to the present disclosure may be used alone or in combination with radiation therapy and/or chemotherapy to exhibit an effective anti-cancer therapeutic effect and may not exhibit toxicity to normal cells, thereby being usefully employed in anti-cancer therapy.

### [Description of Drawings]

FIG. 1 shows the result of confirming the accumulation of ADP-ribose in various solid cancer cells after ADP-ribose treatment on the cells (A: U-87MG, B: H1975, C: AsPC-1, D: Hep G2, E: MDA-MB-231, F: HCT116, and G: Caki-1 cell line).
FIG. 2 is a photomicrograph (A) and a graph (B) each confirming the cancer cell growth inhibition and cell death effect after ADP-ribose treatment on brain cancer (U-87MG) cells.
FIG. 3 is a photomicrograph (A) and a graph (B) confirming the cancer cell growth inhibition and cell death effect after ADP-ribose treatment on lung cancer (H1975) cells.
FIG. 4 is a photomicrograph (A) and a graph (B) confirming the cancer cell growth inhibition and cell death effect after ADP-ribose treatment on pancreatic cancer (AsPC-1) cells.
FIG. 5 is a photomicrograph (A) and a graph (B) confirming the cancer cell growth inhibition and cell death effect after ADP-ribose treatment on liver cancer (Hep G2) cells.
FIG. 6 is a photomicrograph (A) and a graph (B) confirming the cancer cell growth inhibition and cell death effect after ADP-ribose treatment on breast cancer (MDA-MB-231) cells.
FIG. 7 is a photomicrograph (A) and a graph (B) confirming the cancer cell growth inhibition and cell death effect after ADP-ribose treatment on colon cancer (HCT116) cells.
FIG. 8 is a photomicrograph (A) and a graph (B) confirming the cancer cell growth inhibition and cell death effect after ADP-ribose treatment on kidney cancer (Caki-1) cells.
FIG. 9 is a graph confirming the cancer cell death effect after ADP-ribose treatment on gastric cancer (SNU-1) cells or ovarian cancer (OVCAR-3) cells.
FIG. 10 is a photomicrograph (A) and a graph (B) confirming the reduction in cancer cell invasion and metastasis ability after ADP-ribose treatment of pancreatic cancer (AsPC-1) cells.
FIG. 11 is a photomicrograph (A) and a graph (B) confirming the cancer cell invasion and metastasis ability after ADP-ribose treatment on breast cancer (MDA-MB-231) cells.
FIG. 12 is a graph (A) and a photomicrograph (B) showing the effect of reducing tumor volume after subcutaneous injection (S.C) of ADP-ribose in a xenograft animal model induced with a pancreatic cancer cell line.
FIG. 13 is a graph (A) and a photomicrograph (B) showing the effect of reducing tumor volume after intravenous injection (I.V) of ADP-ribose in a xenograft animal model induced with a kidney cancer cell line.
FIG. 14 is a graph (A) and a photomicrograph (B) showing the effect of reducing tumor volume after oral administration (P.O) of ADP-ribose in a xenograft animal model induced with a pancreatic cancer cell line.
FIG. 15 is a graph showing the synergistic cell death effect when various solid tumor cells (A: U-87MG, B: H1975, C: AsPC-1, D: Hep G2, E: MDA-MB-231, and F: HCT116) were treated in combination with low-dose anti-cancer drugs (A: bevacizumab, B: osimertinib, C: gemcitabine, D: sorafenib, E: Herceptin, and F: 5-fluorouracil) and ADP-ribose.
FIG. 16 is a graph showing the synergistic tumor volume reduction effect when tumor-inducing animals were administered in combination with low-dose anti-cancer drugs (A: bevacizumab, B: osimertinib, C: gemcitabine, and D: sorafenib) and ADP-ribose.
FIG. 17 is a graph showing the comparison results of ADP-ribose accumulation in cells when solid cancer (A: U-87MG, B: Caki-1, C: AsPC-1, and D: MDA-MB-231) cells were irradiated with radiation and when treated with ADP-ribose.
FIG. 18 is a graph confirming the synergistic cell death effect when solid cancer (A: U-87MG, B: H1975, C: AsPC-1, D: Hep G2, E: MDA-MB-231, F: HCT116, and G: Caki-1) cells were treated in combination with the irradiation-resistant dose of radiation and ADP-ribose.
FIG. 19 is a graph showing the synergistic tumor volume reduction effect when tumor-inducing animals were administered in combination with low-dose irradiation and ADP-ribose.
FIG. 20 is a photograph and a graph confirming whether cytotoxicity was observed after ADP-ribose was administered to normal cells (A, B: human colon fibroblast, and C, D: human hair dermal papilla cells).

### [Mode for Invention]

Hereinafter, the present disclosure will be described in more detail through Examples. However, these Examples are provided to illustrate the present disclosure by way of example, and the scope of the present disclosure is not limited to these Examples.

### Materials and Methods

### 1. Preparation of ADP-ribose and anti-cancer drug

ADP-ribose and anti-cancer drugs such as bevacizumab, osimertinib, gemcitabine, sorafenib, herceptin, and 5-fluorouracil were all purchased from Sigma-Aldrich (St. Louis, MO, USA). ADP-ribose and all anti-cancer drugs were stored frozen at -20°C until use.

### 2. Preparation of cell lines

Brain cancer (U-87MG), lung cancer (H1975), pancreatic cancer (Aspc-1), liver cancer (Hep G2), breast cancer (MDA-MB-231), colon cancer (HCT116), kidney cancer (Caki-1), gastric cancer (SNU-1) and ovarian cancer (OVCAR-3) cells were purchased from the American Type Culture Collection (ATCC, Manassas, VA, USA) and stored in liquid nitrogen until used for research.

### 3. ADP-ribose quantitative analysis

5 × 10⁵ brain cancer (U-87MG), lung cancer (H1975), pancreatic cancer (Aspc-1), liver cancer (Hep G2), breast cancer (MDA-MB-231), colon cancer (HCT116), and kidney cancer (Caki-1) cells were mixed with 10% fetal calf serum + 100 units/ml penicillin + 100 µg/ml streptomycin in each appropriate medium, and the cells were divided into the untreated group and ADP-ribose-treated groups, and cultured under conditions of 37°C and 5% CO₂ for 24 hours. After culturing, the culture medium was removed, the cells were washed with phosphate-buffered saline, and then RIPA buffer was added and the cells were left on ice for 20 minutes. After scraping the cells, the cells were put in a centrifuge tube and centrifuged for 10 minutes at 10,000 g and 4°C. The supernatant was obtained, and 1% SDS was added, and then the reaction product was boiled at 100°C for 5 minutes and cooled on ice. The supernatant was centrifuged again at 10,000 g and 4°C for 10 minutes to obtain the final supernatant, and ADP-ribose was quantitatively analyzed and compared using an ELISA analysis kit (Cell Biolabs, Inc., San Diego, CA, USA).

### 4. Cancer cell death verification

3 × 10³ Brain cancer (U-87MG), lung cancer (H1975), pancreatic cancer (Aspc-1), liver cancer (Hep G2), breast cancer (MDA-MB-231), colon cancer (HCT116), kidney cancer (Caki-1), gastric cancer (SNU-1) and ovarian cancer (OVCAR-3) cells were cultured in a 96-well plate under conditions of 37°C and 5% CO₂ for 24 hours, and then each cancer cell was treated with ADP-ribose at each concentration. After culturing for 24 hours under conditions of 37°C and 5% CO₂ again, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to determine the concentration of half-lethality and maximum lethality compared to untreated cells.

### 5. Verification of inhibition of metastasis and invasion ability of cancer cell

Metastasis and invasion ability of cancer cells were analyzed by using a transwell having polycarbonate membrane filters with 8.0 um pore size. The transwell was put into a 24-well plate. In each well, pancreatic cancer (Aspc-1) and breast cancer (MDA-MB-231) cells were seeded on the upper surface of the insert at a density of 3.5 × 10⁵ cells per well, divided into the untreated group and ADP-ribose-treated groups, and cultured under conditions of 37°C and 5% CO₂ for 24 hours. After culturing, the transwell was carefully washed with sterile water and then placed in 100% methanol to fix the attached cells. After fixation, the cells were stained with hematoxylin and an eosin reagent to observe the cells, and the ratio of cells that permeated the upper surface of the insert and metastasized and invaded the lower surface, compared to the untreated group, was obtained.

### 6. Preparation of animals

5-week-old BALB/c nude mice were ordered from Charles River Laboratories (Wilmington, MA, USA). The breeding facility was an environment without specific lesions and maintained at a temperature of 22-25°C, the day and night cycle was maintained in a 12-hour cycle (lights on at 8:00 am), and feed and drinking water were provided autonomously. All animal studies were conducted according to the regulations of the Animal Research Ethics Committee (AREC).

### 7. Verification of anti-cancer efficacy according to the administration route and concentration of ADP-ribose in the xenograft model

Pancreatic cancer (AsPC-1) or kidney cancer (Caki-1) cells (1 × 10⁷ cells) were inoculated into 5-week-old BALB/c nude mice on the back flank, and the mice were grown up to approximately 150 mm³ of the tumor volume. Then, three concentrations of ADP-ribose were administered subcutaneously, intravenously, and orally 3 times a week. The tumor size was measured every 6 days using a digital caliper, and the changes in tumor volume were compared for each group.

### 8. Verification of cell death by combination treatment with anti-cancer drug in cancer cell

3 × 10³ Brain cancer (U-87MG), lung cancer (H1975), pancreatic cancer (Aspc-1), liver cancer (Hep G2), breast cancer (MDA-MB-231), and colon cancer (HCT116) cells were cultured in a 96-well plate for 24 hours under conditions of 37°C and 5% CO₂, respectively. Then, the brain cancer (U-87MG) cells were treated with 15 mM bevacizumab, the lung cancer (H1975) cells were treated with 1 nM osimertinib, pancreatic cancer (Aspc-1) cells were treated with 1 µM gemcitabine, the liver cancer (Hep G2) cells were treated with 1 µM sorafenib, the breast cancer (MDA-MB-231) cells were treated with 2.5 µM herceptin, and the colon cancer (HCT116) cells were treated with 10 µM 5-fluorouracil alone or in combination with ADP-ribose. After the treatment was terminated, the cells were cultured for 24 hours under conditions of 37°C and 5% CO₂ again, 10 µl of 3-(4, 5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to compare the degree of cell death.

### 9. Verification of anti-cancer efficacy according to the combination of anti-cancer drugs in the xenograft model

Brain (U-87MG), lung (H1975), pancreatic (AsPC-1), or liver (Hep G2) cells (1 × 10⁷ cells) were inoculated into 5-week-old BALB/c nude mice on the back flank, respectively. Then, when tumors were grown to a volume of approximately 150 mm³, the mice were divided into the control group (control), an anti-cancer drug alone treatment group, and a combination treatment group. To mice, for the brain cancer (U-87MG) study, 25 mg/kg low-dose of bevacizumab was administered by intraperitoneal injection twice a week; for lung cancer (H1975) study, 1 mg/kg low-dose of osimertinib was administered by oral injection twice a week; for pancreatic cancer (AsPC-1) study, 50 mg/kg low-dose gemcitabine was administered by intraperitoneal injection twice a week; and for liver cancer (Hep G2) study, 10 mg/kg low dose of sorafenib was administered by oral injection 5 times a week either alone or in combination with each of the above-described anti-cancer drugs and 10 mg/kg of ADP-ribose (subcutaneous administration, 3 times a week). The tumor size was measured every 6 days using a digital caliper, and the results of changes in tumor volume were compared by the group.

### 10. Quantitative analysis of ADP-ribose according to the combination treatment of radiation in cancer cells

Brain cancer (U-87MG), kidney cancer (Caki-1), pancreatic cancer (AsPC-1), or breast cancer (MDA-MB-231) cells were cultured in a 6-well plate, respectively, and the cells were divided into the untreated group, the radiation-treated group, and the ADP-ribose-treated group. Irradiation was performed using an X-Rad 320 irradiator (Precision X-ray, North Branford, CT, USA), and cancer cells were treated with a total of 1, 2, and 5 Gy at a dose rate of 300 kVp and 150 cGy/min using an X-ray beam filter composed of 12.5 mA and 2.0 mm Al, respectively. Before treatment and at 4, 8, 16, and 24 hours after treatment, the medium was removed, the cells were washed with phosphate-buffered saline, and then RIPA buffer was added and the products were left on ice for 20 minutes. After scraping the cells, the cells were put in a centrifuge tube and centrifuged for 10 minutes at 10,000 g and 4°C. The supernatant was obtained, and 1% SDS was added, and then the reaction product was boiled at 100°C for 5 minutes and cooled on ice. The supernatant was centrifuged again at 10,000 g and 4°C for 10 minutes to obtain the final supernatant, and ADP-ribose was quantitatively analyzed using an ELISA analysis kit (Cell Biolabs, Inc., San Diego, CA, USA).

### 11. Verification of cell death according to the combination treatment of radiation in cancer cells

3 × 10³ Brain cancer (U-87MG), lung cancer (H1975), pancreatic cancer (Aspc-1), liver cancer (Hep G2), breast cancer (MDA-MB-231), colon cancer (HCT116), and kidney cancer (Caki-1) cells were cultured in a 96-well plate for 24 hours under conditions of 37°C and 5% CO₂, treated with ADP-ribose or 2 or 5 Gy radiation alone or in combination with ADP-ribose and 2 Gy radiation, and cultured for 24 hours under conditions of 37°C and 5% CO₂ again. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to compare the degree of cell death.

### 12. Verification of anti-cancer efficacy according to the combination treatment of radiation in the xenograft model

Brain cancer (U-87MG), pancreatic cancer (AsPC-1), breast cancer (MDA-MB-231), or kidney cancer (Caki-1) cells (1 × 10⁷ cells) were inoculated into 5-week-old BALB/c nude mice on the back flank, respectively. Then, when tumors were grown to a volume of approximately 150 mm³, the mice were divided into three groups: the control group (Control), 2 Gy dose of radiation alone, and a combination administration of 2 Gy dose of radiation + 10 mg/kg of ADP-ribose (subcutaneous administration, 3 times a week). The irradiation was performed with an X-Rad 320 irradiator (Precision X-ray, North Branford, CT, USA), wherein the mice were placed by maintaining an appropriate distance from the radiation source for irradiation with a predetermined radiation dose. A lead shield was used to protect the body parts or organs from exposure to radiation, other than the cancerous part of the mouse. The irradiation was performed with a total of 2 Gy of radiation fractionated into two doses of 1 Gy each (for 2 days) at a dose rate of 300 kVp and 150 cGy/min using an X-ray beam filter composed of 12.5 mA and 2.0 mm Al. The tumor size was measured every 6 days using a digital caliper, and the results of changes in tumor volume were compared by the group.

### 13. Statistical significance analysis

All graphs are presented as mean ± standard deviation values. Statistical significance was analyzed using Student's t-test analysis according to whether data were normalized or not. A difference of P<0.05 or more was considered statistically significant, and Systat Software's program was used (Sigmastat ver. 3.5, Systat Software Inc., Chicago, IL, USA) for statistical significance analysis.

### Example 1: Change in ADP-ribose level in cancer cells

### Example 1-1: Change in ADP-ribose level in U-87MG cells

5 × 10⁵ U-87MG cells were divided into the untreated group and 8 µM ADP-ribose-treated group and cultured in Eagle's Minimum Essential Medium (EMEM) supplemented with 10% Fetal calf serum + 100 units/ml penicillin + 100 µg/ml streptomycin for 24 hours under conditions of 37°C and 5% CO₂. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when brain cancer cells (U-87MG) were treated with ADP-ribose, the amount of ADP-ribose remarkably increased significantly (P<0.001) compared to the control group (FIG. 1A).

### Example 1-2: Change in ADP-ribose level in H1975 cells

5 × 10⁵ H1975 cells were divided into the untreated group and 4 µM ADP-ribose-treated group and cultured in RPMI-1640 medium supplemented with 10% fetal calf serum + 100 units/ml penicillin + 100 µg/ml streptomycin for 24 hours under conditions of 37°C and 5% CO₂. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when lung cancer cells (H1975) were treated with ADP-ribose, the amount of ADP-ribose remarkably increased significantly (P<0.001) compared to the control group (FIG. 1B).

### Example 1-3: Change in ADP-ribose level in AsPC-1 cells

5 × 10⁵ AsPC-1 cells were divided into the untreated group and 8 µM ADP-ribose-treated group and cultured in RPMI-1640 medium supplemented with 10% fetal calf serum + 100 units/ml penicillin + 100 µg/ml streptomycin for 24 hours under conditions of 37°C and 5% CO₂. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when pancreatic cancer cells (AsPC-1) were treated with ADP-ribose, the amount of ADP-ribose remarkably increased significantly (P<0.001) compared to the control group (FIG. 1C).

### Example 1-4: Change in ADP-ribose level in Hep G2 cells

5 × 10⁵ Hep G2 cells were divided into the untreated group and 8 µM ADP-ribose-treated group and cultured in Eagle's Minimum Essential Medium supplemented with 10% fetal calf serum + 100 units/ml penicillin + 100 µg/ml streptomycin under conditions of 37°C and 5% CO₂ for 24 hours. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when liver cancer cells (Hep G2) were treated with ADP-ribose, the amount of ADP-ribose remarkably increased significantly (P<0.001) compared to the control group (FIG. 1D).

### Example 1-5: Change in ADP-ribose level in MDA-MB-231 cells

5 × 10⁵ MDA-MB-231 cells were divided into the untreated group and 16 µM ADP-ribose-treated group and cultured in Leibovitz's L-15 medium supplemented with 10% fetal calf serum + 100 units/ml penicillin + 100 µg/ml streptomycin under conditions of 37°C and 5% CO₂ for 24 hours. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when breast cancer cells (MDA-MB-231) were treated with ADP-ribose, the amount of ADP-ribose remarkably increased significantly (P<0.001) compared to the control group (FIG. 1E).

### Example 1-6: Change in ADP-ribose level in HCT116 cell

5 × 10⁵ HCT116 cells were divided into the untreated group and 8 µM ADP-ribose-treated group and cultured in McCoy's 5A medium supplemented with 10% fetal calf serum + 100 units/ml penicillin + 100 µg/ml streptomycin for 24 hours under conditions of 37°C and 5% CO₂. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when colon cancer cells (HCT116) were treated with ADP-ribose, the amount of ADP-ribose remarkably increased significantly (P<0.001) compared to the control group (FIG. 1F).

### Example 1-7: Change in ADP-ribose level in Caki-1 cell

5 × 10⁵ Caki-1 cells were divided into the untreated group and 16 µM ADP-ribose-treated group and cultured in McCoy's 5A medium supplemented with 10% fetal calf serum + 100 units/ml penicillin + 100 µg/ml streptomycin for 24 hours under conditions of 37°C and 5% CO₂. The medium of the cultured cells was removed, and the supernatant prepared by treatment with RIPA buffer and 1% sodium dodecyl sulfate (SDS) was used for ELISA analysis to detect and analyze ADP-ribose.

As a result, it was confirmed that when kidney cancer cells (Caki-1) were treated with ADP-ribose, the amount of ADP-ribose remarkably increased significantly (P<0.001) compared to the control group (FIG. 1G).

Summarizing the above, Example 1 showed that the intracellular ADP-ribose concentration could be significantly increased by external ADP-ribose treatment in various solid cancer (brain cancer, lung cancer, pancreatic cancer, liver cancer, breast cancer, colon cancer, and kidney cancer) cells (FIG. 1).

### Example 2: Change in cancer cell viability depending on ADP-ribose concentration

### Example 2-1: Change in viability in U-87MG cells according to increase in ADP-ribose concentration

3 × 10³ U-87MG cells were cultured in a 96-well plate for 24 hours under conditions of 37°C and 5% CO₂, treated with ADP-ribose at final concentrations of 0.125, 0.25, 0.5, 1, 2, 4, 8, 16, and 32 µM, respectively, and cultured again for 24 hours under conditions of 37°C and 5% CO₂. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed under a microscope that the rapid growth of brain cancer cells (U-87MG) was observed in the control group not treated with ADP-ribose, whereas the experimental group treated with ADP-ribose showed that the brain cancer cells were inhibited from growing and killed (FIG. 2A). The half-lethal dose and the maximum lethal dose according to the ADP-ribose treatment concentration were quantitatively measured, and as a result, it was confirmed that about half of the brain cancer cells were killed at a concentration of 8 µM, and almost all cells were killed at 32 µM (FIG. 2B).

### Example 2-2: Change in viability in H1975 cells according to increase in ADP-ribose concentration

3 × 10³ H1975 cells were cultured in a 96-well plate for 24 hours under conditions of 37°C and 5% CO₂, treated with ADP-ribose at concentrations of 0.125, 0.25, 0.5, 1, 2, 4, 8, 16, and 32 µM, respectively, and cultured again for 24 hours under conditions of 37°C and 5% CO₂. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

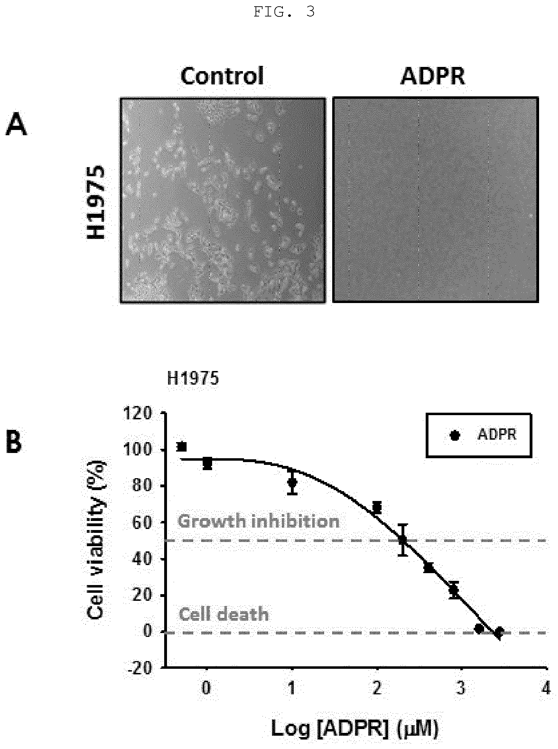

As a result, it was confirmed under a microscope that the rapid growth of lung cancer cells (H1975) was observed in the control group not treated with ADP-ribose, whereas the experimental group treated with ADP-ribose showed that the lung cancer cells were inhibited from growing and killed (FIG. 3A). The half-lethal dose and the maximum lethal dose according to the ADP-ribose treatment concentration were quantitatively measured, and as a result, it was confirmed that about half of the lung cancer cells were killed at a concentration of 2 µM, and almost all cells were killed at 32 µM (FIG. 3B).

### Example 2-3: Change in viability in AsPC-1 cells according to increase in ADP-ribose concentration

3 × 10³ AsPC-1 cells were cultured in a 96-well plate for 24 hours under conditions of 37°C and 5% CO₂, treated with ADP-ribose at concentrations of 0.125, 0.25, 0.5, 1, 2, 4, 8, 16, and 32 µM, respectively, and cultured again for 24 hours under conditions of 37°C and 5% CO₂. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

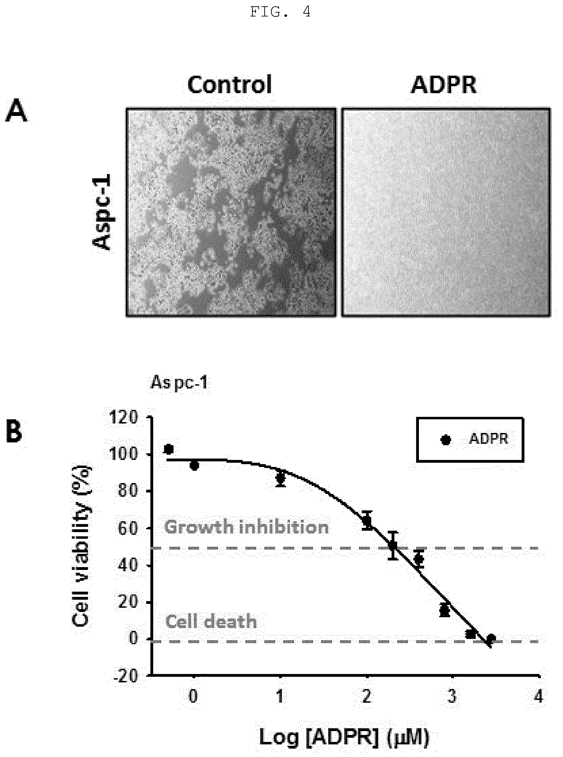

As a result, it was confirmed under a microscope that the rapid growth of pancreatic cancer cells (AsPC-1) was observed in the control group not treated with ADP-ribose, whereas the experimental group treated with ADP-ribose showed that the pancreatic cancer cells were inhibited from growing and killed (FIG. 4A). The half-lethal dose and the maximum lethal dose according to the ADP-ribose treatment concentration were quantitatively measured, and as a result, it was confirmed that about half of the pancreatic cancer cells were killed at a concentration of 2 µM, and almost all cells were killed at 32 µM (FIG. 4B).

### Example 2-4: Change in viability in Hep G2 cells according to increase in ADP-ribose concentration

3 × 10³ Hep G2 cells were cultured in a 96-well plate for 24 hours under conditions of 37°C and 5% CO₂, treated with ADP-ribose at concentrations of 0.125, 0.25, 0.5, 1, 2, 4, 8, 16, and 32 µM, respectively, and cultured again for 24 hours under conditions of 37°C and 5% CO₂. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

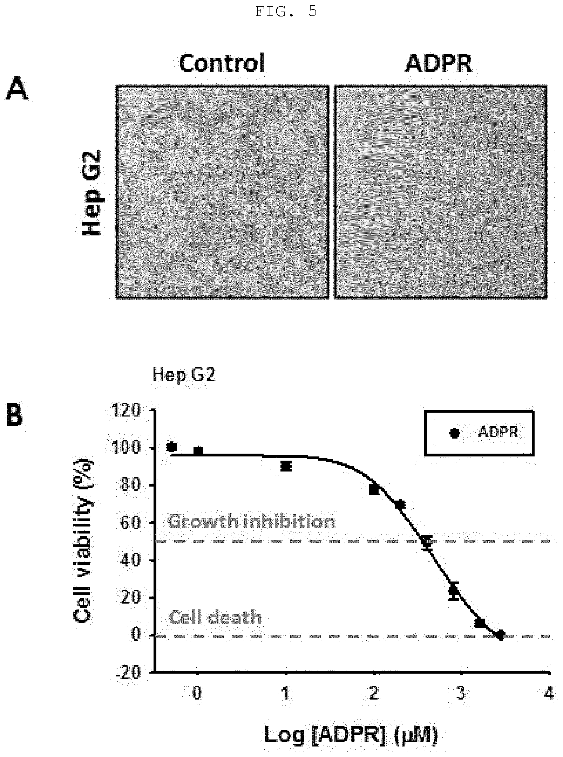

As a result, it was confirmed under a microscope that the rapid growth of liver cancer cells (Hep G2) was observed in the control group not treated with ADP-ribose, whereas the experimental group treated with ADP-ribose showed that the liver cancer cells were inhibited from growing and killed (FIG. 5A). The half-lethal dose and the maximum lethal dose according to the ADP-ribose treatment concentration were quantitatively measured, and as a result, it was confirmed that about half of the liver cancer cells were killed at a concentration of 4 µM, and almost all cells were killed at 32 µM (FIG. 5B).

### Example 2-5: Change in viability in MDA-MB-231 cells according to increase in ADP-ribose concentration

3 × 10³ MDA-MB-231 cells were cultured in a 96-well plate for 24 hours under conditions of 37°C and 5% CO₂, treated with ADP-ribose at concentrations of 0.125, 0.25, 0.5, 1, 2, 4, 8, 16, and 32 µM, respectively, and cultured again for 24 hours under conditions of 37°C and 5% CO₂. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

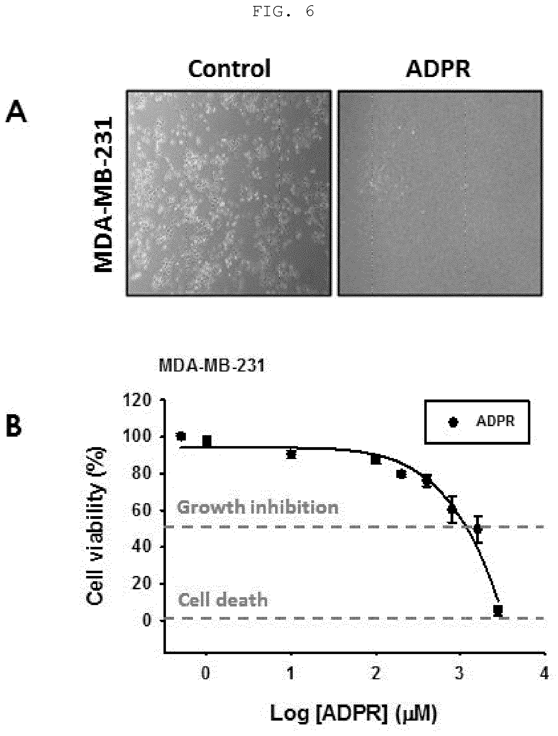

As a result, it was confirmed under a microscope that the rapid growth of breast cancer cells (MDA-MB-231) was observed in the control group not treated with ADP-ribose, whereas the experimental group treated with ADP-ribose showed that the breast cancer cells were inhibited from growing and killed (FIG. 6A). The half-lethal dose and the maximum lethal dose according to the ADP-ribose treatment concentration were quantitatively measured, and as a result, it was confirmed that about half of the breast cancer cells were killed at a concentration of 16 µM, and almost all cells were killed at 32 µM (FIG. 6B).

### Example 2-6: Change in viability in HCT116 cells according to increase in ADP-ribose concentration

3 × 10³ HCT116 cells were cultured in a 96-well plate for 24 hours under conditions of 37°C and 5% CO₂, treated with ADP-ribose at concentrations of 0.125, 0.25, 0.5, 1, 2, 4, 8, 16, and 32 µM, respectively, and cultured again for 24 hours under conditions of 37°C and 5% CO₂. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

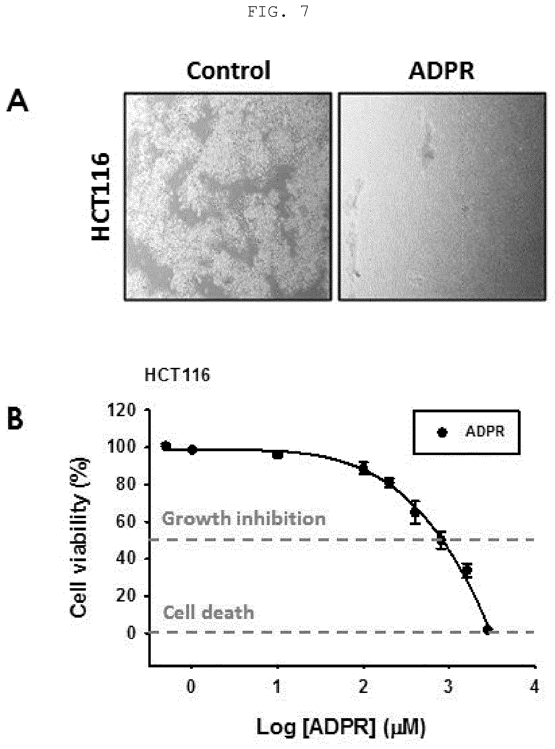

As a result, it was confirmed under a microscope that the rapid growth of colon cancer cells (HCT116) was observed in the control group not treated with ADP-ribose, whereas the experimental group treated with ADP-ribose showed that the colon cancer cells were inhibited from growing and killed (FIG. 7A). The half-lethal dose and the maximum lethal dose according to the ADP-ribose treatment concentration were quantitatively measured, and as a result, it was confirmed that about half of the colon cancer cells were killed at a concentration of 8 µM, and almost all cells were killed at 32 µM (FIG. 7B).

### Example 2-7: Change in viability in Caki-1 cells according to increase in ADP-ribose concentration

3 × 10³ Caki-1 cells were cultured in a 96-well plate for 24 hours under conditions of 37°C and 5% CO₂, treated with ADP-ribose at concentrations of 0.125, 0.25, 0.5, 1, 2, 4, 8, 16, and 32 µM, respectively, and cultured again for 24 hours under conditions of 37°C and 5% CO₂. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

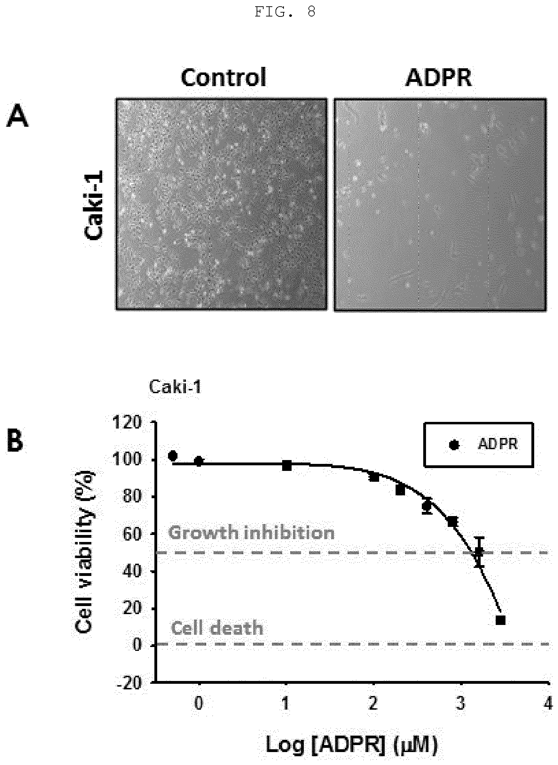

As a result, it was confirmed under a microscope that the rapid growth of kidney cancer cells (Caki-1) was observed in the control group not treated with ADP-ribose, whereas the experimental group treated with ADP-ribose showed that the kidney cancer cells were inhibited from growing and killed (FIG. 8A). The half-lethal dose and the maximum lethal dose according to the ADP-ribose treatment concentration were quantitatively measured, and as a result, it was confirmed that about half of the kidney cancer cells were killed at a concentration of 16 µM, and almost all cells were killed at 32 µM (FIG. 8B).

### Example 2-8: Change in viability in SNU-1 or OVCAR-3 cells according to an increase in ADP-ribose concentration

3 × 10³ SNU-1 or OVCAR-3 cells were cultured in a 96-well plate for 24 hours under conditions of 37°C and 5% CO₂, treated with ADP-ribose at concentrations of 32 µM, respectively, and cultured again for 24 hours under conditions of 37°C and 5% CO₂. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

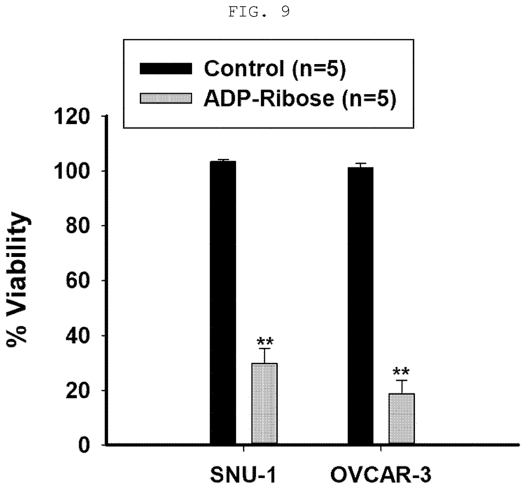

As a result, it was confirmed that when gastric cancer cells (SNU-1) or ovarian cancer cells (OVCAR-3) were treated with ADP-ribose, the cells were killed significantly compared to the control group (FIG. 9).

Summarizing the above, it was confirmed through the cell experiment of Example 2 that ADP-ribose had an anti-cancer effect on cells of various solid cancers (brain cancer, lung cancer, pancreatic cancer, liver cancer, breast cancer, colon cancer, kidney cancer, gastric cancer, and ovarian cancer) (FIGS. 2 to 9).

### Example 3: Change in metastasis and invasion ability of metastatic cancer cells by ADP-ribose

When the growth of cancer cells is inhibited and death is induced, the ability of cancer cells to metastasize or invade may also be affected. As in Example 2, the effects of inhibiting the growth of cancer cells and inducing cell death were clearly observed by treatment with ADP-ribose. Thus, it is possible to expect that the treatment with ADP-ribose may induce a reduction in metastasis and invasion ability of cancer cells.

### Example 3-1: Change in metastasis and invasion ability of AsPC-1 cells by ADP-ribose

Analysis was performed using a transwell having polycarbonate membrane filters with 8.0 um pore size. The transwell was put in a 24-well plate, and AsPC-1 cells were seeded on the upper surface of the insert at a density of 3.5 × 10⁵ cells per well, and cultured for 24 hours under conditions of 37°C and 5% CO₂. After culturing, the transwell was carefully washed with sterile water and then placed in 100% methanol to fix the attached cells. After fixation, the cells were stained with hematoxylin and an eosin reagent to observe the cells.

As a result, it was confirmed that invasion and metastasis of pancreatic cancer cells (AsPC-1) from the upper part to the lower part of the transwell were easily observed in the control group, whereas the experimental group treated with 16 µM ADP-ribose showed that the invasion and metastasis of pancreatic cancer cells were significantly inhibited (FIG. 10A). The metastasis and invasion inhibitory effect of ADP-ribose on pancreatic cancer cells were quantitatively measured, and as a result, it was confirmed that the metastasis and invasion ability were significantly reduced (P<0.001) compared to the control group (FIG. 10B).

### Example 3-2: Change in metastasis and invasion ability of MDA-MB-231 cells by ADP-ribose

Analysis was performed using a transwell having polycarbonate membrane filters with 8.0 um pore size. The transwell was put in a 24-well plate, and MDA-MB-231 cells were seeded on the upper surface of the insert at a density of 3.5 × 10⁵ cells per well, and cultured for 24 hours under conditions of 37°C and 5% CO₂. After culturing, the transwell was carefully washed with sterile water and then placed in 100% methanol to fix the attached cells. After fixation, the cells were stained with hematoxylin and an eosin reagent to observe the cells.

As a result, it was confirmed that invasion and metastasis of breast cancer cells (MDA-MB-231) from the upper part to the lower part of the transwell were easily observed in the control group, whereas the experimental group treated with 32 µM ADP-ribose showed that the invasion and metastasis of breast cancer cells were significantly inhibited (FIG. 11A). The metastasis and invasion inhibitory effect of ADP-ribose on breast cancer cells were quantitatively measured, and as a result, it was confirmed that the metastasis and invasion ability were significantly reduced (P<0.001) compared to the control group (FIG. 11B).

Summarizing the above, it was confirmed in Example 3 that ADP-ribose inhibited the metastasis and invasion ability of solid cancer (pancreatic cancer, breast cancer) cells, thereby providing excellent therapeutic effects on metastatic cancer (FIGS. 10 and 11).

### Example 4: Comparison and verification of anti-cancer efficacy according to the concentration and administration route of ADP-ribose using animal models

### Example 4-1: Change in tumor volume in animal models injected subcutaneously with various concentrations of ADP-ribose

Pancreatic cancer (AsPC-1) cells (1 × 10⁷) were inoculated into 5-week-old BALB/c nude mice on the back flank, and the mice were divided into four groups: the control group (Control) and groups injected subcutaneously with 2, 20, and 40 mg/kg of ADP-ribose. When the tumors were grown to a volume of approximately 150 mm3, each concentration of ADP-ribose was subcutaneously injected 3 times a week. The tumor size was measured using a digital caliper, and the results of changes in tumor volume were compared by the group.

As a result of measuring the volume of pancreatic cancer injected over time, the final volume of tumors in the control group after the last dose was about 2155.8 mm³, and the final tumor volumes of the groups injected subcutaneously with 2, 20, and 40 mg/kg of ADP-ribose were 1015.8 mm3, 496.6 mm3, and 336.2 mm³, respectively. In other words, in all administration groups, tumor volume was reduced statistically significantly (P<0.001) compared to the control group. Specifically, compared to the group injected subcutaneously with 2 mg/kg of ADP-ribose, the group injected with 20 mg/kg of ADP-ribose had a significant reduction (P<0.001) in tumor volume, and the group injected with 40 mg/kg of ADP-ribose subcutaneously had the smallest tumor volume and showed a statistically significant decrease (P<0.001) as compared to the group injected subcutaneously with 2 mg/kg ADP-ribose (FIGS. 12A and 12B).

### Example 4-2: Change in tumor volume in animal models injected intravenously with various concentrations of ADP-ribose

Kidney cancer (Caki-1) cells (1 × 10⁷) were inoculated into 5-week-old BALB/c nude mice on the back flank, and the mice were divided into 4 groups: the control group (Control) and groups injected intravenously with 2, 10, and 20 mg/kg of ADP-ribose. When the tumors were grown to a volume of approximately 150 mm³, each concentration of ADP-ribose was subcutaneously injected 3 times a week. The tumor size was measured using a digital caliper, and the results of changes in tumor volume were compared by the group.

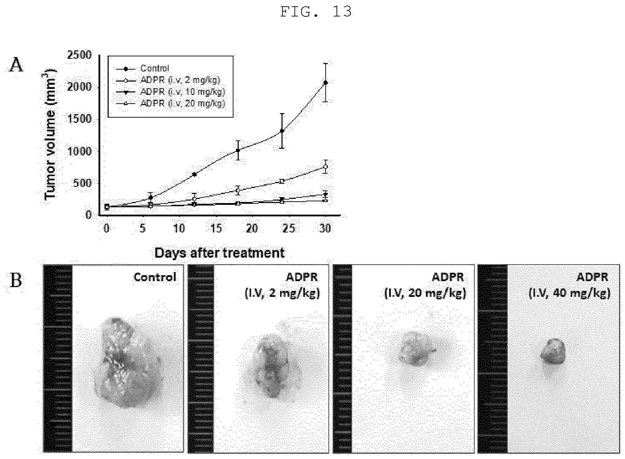

As a result of measuring the volume of the kidney cancer injected over time, the final volume of tumors in the control group after the last dose was about 2074.9 mm3, and the final tumor volumes of the groups injected intravenously with 2, 10, and 20 mg/kg of ADP-ribose were 706.7 mm3, 331.7 mm3, and 227.2 mm3, respectively. In other words, in all injection groups, tumor volume was reduced statistically significantly (P<0.001) compared to the control group. Specifically, compared to the group injected intravenously with 2 mg/kg of ADP-ribose, the group injected intravenously with 10 mg/kg of ADP-ribose had a significant reduction (P<0.001) in tumor volume, and the group injected intravenously with 20 mg/kg of ADP-ribose had the smallest tumor volume and showed a statistically significant decrease (P<0.001) as compared to the groups injected intravenously with 2 mg/kg ADP-ribose and with 10 mg/kg ADP-ribose (FIGS. 13A and 13B).

### Example 4-3: Change in tumor volume in animal models administered orally with various concentrations of ADP-ribose

Pancreatic cancer (AsPC-1) cells (1 × 10⁷) were inoculated into 5-week-old BALB/c nude mice on the back flank, and the mice were divided into four groups: the control group (Control) and groups administered orally with 10, 40, and 80 mg/kg of ADP-ribose. When the tumors were grown to a volume of approximately 150 mm3, each concentration of ADP-ribose was orally administered 5 times a week. The tumor size was measured using a digital caliper, and the results of changes in tumor volume were compared by the group.

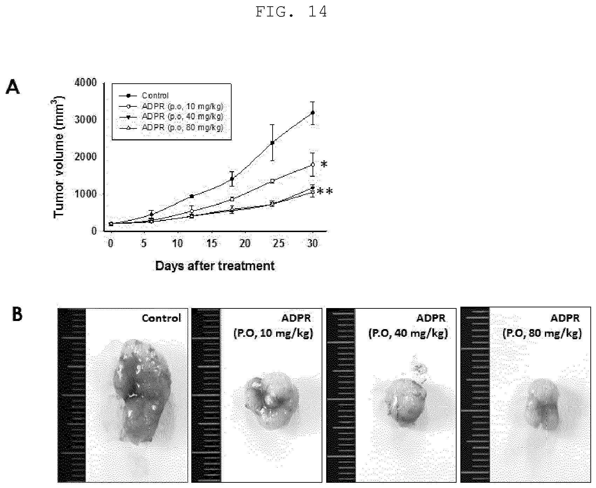

As a result of measuring the volume of pancreatic cancer injected over time, the final volume of tumors in the control group (Control) after the last dose was about 3194.3 mm³, and the final tumor volumes of the groups injected orally with 10, 40, and 80 mg/kg of ADP-ribose were 1798.6 mm³, 1175.9 mm3, and 1056.9 mm3, respectively. In other words, in all oral administration groups, tumor volume was reduced statistically significantly (P<0.001) compared to the control group. Compared to the group administered orally with 10 mg/kg of ADP-ribose, the tumor volume of the groups injected orally with 40 and 80 mg/kg of ADP-ribose was significantly reduced (P<0.001) (FIGS. 14A and 14B).

Summarizing the above, it was reconfirmed that ADP-ribose had an anti-cancer effect against various solid cancers through the animal experiment of Example 4, which proceeded after Examples 2 and 3 showing the cell experiment results (FIGS. 12 to 14).

### Example 5: Change in cancer cell viability by combination treatment of ADP-ribose and low-dose anti-cancer drug

### Example 5-1: Change in viability in U-87MG cells by the combination treatment of ADP-ribose and low-dose bevacizumab

3 × 10³ Brain cancer (U-87MG) cells were cultured in a 96-well plate for 24 hours under conditions of 37°C and 5% CO₂, treated with ADP-ribose at a concentration of 1.6 µM and bevacizumab at 15 mM, individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when brain cancer cells were treated with bevacizumab alone, about 68% of all cancer cells survived, but when treated in combination with ADP-ribose, the viability of cancer cells was significantly reduced to about 18% (P<0.001) (FIG. 15A).

### Example 5-2: Change in viability in H1975 cells by combination treatment of ADP-ribose and low-dose osimertinib

3 × 10³ Lung cancer (H1975) cells were cultured in a 96-well plate for 24 hours under conditions of 37°C and 5% CO₂, treated with ADP-ribose at a concentration of 1.6 µM and osimertinib at 1 nM, individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when lung cancer cells were treated with osimertinib alone, about 50% of all cancer cells survived, but when treated in combination with ADP-ribose, the viability of cancer cells was significantly reduced to about 0.9% (P<0.001) (FIG. 15B).

### Example 5-3: Change in viability in AsPC-1 cells by combination treatment of ADP-ribose and low-dose gemcitabine

3 × 10³ Pancreatic cancer (AsPC-1) cells were cultured in a 96-well plate for 24 hours under conditions of 37°C and 5% CO₂, treated with ADP-ribose at a concentration of 1.6 µM and gemcitabine at 1 µM, individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when pancreatic cancer cells were treated with gemcitabine alone, about 54% of all cancer cells survived, but when treated in combination with ADP-ribose, the viability of cancer cells was significantly reduced to about 1% (P<0.001) (FIG. 15C).

### Example 5-4: Change in viability in Hep G2 cells by combination treatment of ADP-ribose and low-dose sorafenib

3 × 10³ Liver cancer (Hep G2) cells were cultured in a 96-well plate for 24 hours under conditions of 37°C and 5% CO₂, treated with ADP-ribose at a concentration of 1.6 µM and sorafenib at 1 µM, individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when liver cancer cells were treated with sorafenib alone, about 49% of all cancer cells survived, but when treated in combination with ADP-ribose, the viability of cancer cells was significantly reduced to about 0.9% (P<0.001) (FIG. 15D).

### Example 5-5: Change in viability in MDA-MB-231 cells by combination treatment of ADP-ribose and low-dose herceptin

3 × 10³ Breast cancer (MDA-MB-231) cells were cultured in a 96-well plate for 24 hours under conditions of 37°C and 5% CO₂, treated with ADP-ribose at a concentration of 3.2 µM and herceptin at 2.5 µM, individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when breast cancer cells were treated with 2.5 µM of herceptin alone, as many as 81% of all cancer cells survived due to herceptin resistance, but when treated in combination with ADP-ribose, the viability of cancer cells was significantly reduced to about 20% (P<0.001) (FIG. 15E).

### Example 5-6: Change in viability in HCT116 cells by combination treatment of ADP-ribose and low-dose 5-fluorouracil

3 × 10³ Colon cancer (HCT116) cells were cultured in a 96-well plate for 24 hours under conditions of 37°C and 5% CO₂, treated with ADP-ribose at a concentration of 1.6 µM/100 µl and 5-fluorouracil (5-FU) at 10 µM, individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when colon cancer cells were treated with 10 µM of 5-FU alone, about 51% of all cancer cells survived, but when treated in combination with ADP-ribose, the viability of cancer cells was significantly reduced to about 12% (P<0.001) (FIG. 15F).

Summarizing the above, it could be appreciated from Example 5 that when administered in combination with ADP-ribose and existing anti-cancer drugs, a synergistic anti-cancer effect on solid cancer was shown, and ADP-ribose could be employed not only as an anti-cancer drug for solid cancer, but also be usefully employed as an anti-cancer adjuvant for existing anti-cancer drug (FIG. 15).

### Example 6: Change in tumor volume in animal models by combination administration of ADP-ribose and low-dose anti-cancer drug

### Example 6-1: Change in tumor volume in brain cancer animal models by combination administration of ADP-ribose and low-dose bevacizumab

Brain cancer (U-87MG) cells (1 × 10⁷) were inoculated into 5-week-old BALB/c nude mice on the back flank, and the mice were divided into three groups: the control group (Control), single intraperitoneal administration of 25 mg/kg of Avastin (ingredient name: bevacizumab), and combination administration of Avastin + 10 mg/kg of ADP-ribose. When the tumors were grown to a volume of approximately 150 mm3, Avastin was administered intraperitoneally twice a week, and ADP-ribose at 10 mg/kg was injected subcutaneously 3 times a week. The tumor size was measured using a digital caliper, and the results of changes in tumor volume were compared by the group.

As a result of measuring and comparing the volume of brain cancer over time, the final volume of tumors in the control group (Control) after the last dose was about 2527.1 mm³, and the final tumor volume of the Avastin-administered group was about 1598.9 mm³, which was about 37% lower than that of the control group, whereas the final tumor volume of the group after combination treatment of Avastin and ADP-ribose was about 334.5 mm3, which showed a significant reduction of about 87% (P<0.001) compared to the control group, and even compared to the group treated with Avastin alone, the tumor volume of the combination treatment group was significantly reduced by about 79% (P<0.001) (FIG. 16A).

### Example 6-2: Change in tumor volume in lung cancer animal models by combination administration of ADP-ribose and low-dose osimertinib

Lung cancer (H1975) cells (1×10⁷) were inoculated into 5-week-old BALB/c nude mice on the back flank, and the mice were divided into three groups: the control group (Control), single oral administration of 1 mg/kg of osimertinib, and combination administration of osimertinib + 10 mg/kg of ADP-ribose. When the tumors were grown to a volume of approximately 150 mm3, osimertinib was administered orally twice a week, and ADP-ribose at 10 mg/kg was injected subcutaneously 3 times a week. The tumor size was measured using a digital caliper, and the results of changes in tumor volume were compared by the group.

As a result of measuring and comparing the volume of lung cancer over time, the final volume of tumors in the control group (Control) after the last dose was about 2265.5 mm3, and the final tumor volume of the osimertinib-administered group was about 1881.9 mm³, which was about 17% lower than that of the control group, whereas the final tumor volume of the group after combination treatment of osimertinib and ADP-ribose was about 424.7 mm3, which showed a significant reduction of about 81% (P<0.001) compared to the control group, and even compared to the group treated with osimertinib alone, the tumor volume of the combination treatment group was significantly reduced by about 77% (P<0.001) (FIG. 16B).

### Example 6-3: Change in tumor volume in pancreatic cancer animal models by combination administration of ADP-ribose and low-dose gemcitabine

Pancreatic cancer (AsPC-1) cells (1×10⁷) were inoculated into 5-week-old BALB/c nude mice on the back flank, and the mice were divided into three groups: the control group (Control), single intraperitoneal administration of 50 mg/kg of gemcitabine, and combination administration of gemcitabine + 10 mg/kg of ADP-ribose. When the tumors were grown to a volume of approximately 150 mm³, gemcitabine was administered intraperitoneally twice a week, and ADP-ribose at 10 mg/kg was injected subcutaneously 3 times a week. The tumor size was measured using a digital caliper, and the results of changes in tumor volume were compared by the group.

As a result of measuring and comparing the volume of pancreatic cancer over time, the final volume of tumors in the control group (Control) after the last dose was about 2174.3 mm³, and the final tumor volume of the gemcitabine-administered group was about 1815.3 mm3, which was about 17% lower than that of the control group, whereas the final tumor volume of the group after combination treatment of gemcitabine and ADP-ribose was about 444.3 mm3, which showed a significant reduction of about 80% (P<0.001) compared to the control group, and even compared to the group treated with gemcitabine alone, the tumor volume of the combination treatment group was significantly reduced by about 76% (P<0.001) (FIG. 16C).

### Example 6-4: Change in tumor volume in liver cancer animal models by combination administration of ADP-ribose and low-dose sorafenib

Liver cancer (Hep G2) cells (1×10⁷) were inoculated into 5-week-old BALB/c nude mice on the back flank, and the mice were divided into three groups: the control group (Control), single oral administration of 10 mg/kg of sorafenib, and combination administration of sorafenib + 10 mg/kg of ADP-ribose. When the tumors were grown to a volume of approximately 150 mm³, sorafenib was administered orally 5 times a week, and ADP-ribose at 10 mg/kg was injected subcutaneously 3 times a week. The tumor size was measured using a digital caliper, and the results of changes in tumor volume were compared by the group.

As a result of measuring and comparing the volume of liver cancer over time, the final volume of tumors in the control group after the last dose was about 2322.4 mm3, and the final tumor volume of the sorafenib-administered group was about 1849.8 mm3, which was about 20% lower than that of the control group, whereas the final tumor volume of the group after combination treatment of sorafenib and ADP-ribose was about 546 mm³, which showed a significant reduction of about 76% (P<0.001) compared to the control group, and even compared to the group treated with sorafenib alone, the tumor volume of the combination treatment group was significantly reduced by about 70% (P<0.001) (FIG. 16D).

Summarizing the above, it could be appreciated from the animal experiment of Example 6, which proceeded after the cell experiment of Example 5, that when administered in combination with ADP-ribose and existing anti-cancer drugs, a synergistic anti-cancer effect on solid cancer was shown even when the anti-cancer drug was administered at a low-dose, and ADP-ribose could be employed not only as an anti-cancer drug for solid cancer, but also be usefully employed as an anti-cancer adjuvant for existing anti-cancer drug (FIG. 16).

### Example 7: Change in cancer cells of ADP-ribose by radiation and ADP-ribose treatment

### Example 7-1: Change over time in ADP-ribose by treatment with various doses of radiation and ADP-ribose in U-87MG cells

U-87MG cells were divided into five groups: no treatment group (Control group), groups treated with 1, 2, and 5 Gy of radiation, and ADP-ribose treatment group, and cultured in a 6-well plate for 0, 4, 8, 16, and 24 hours, respectively. Then, the medium was removed, and the supernatant was prepared by treatment with RIPA buffer and 1% sodium dodecyl sulfate (SDS). The prepared supernatant was used for ELISA analysis for the detection of ADP-ribose.

FIG. 17A is a graph showing the intracellular increase (%) in ADP-ribose according to increasing radiation doses of 1, 2, and 5 Gy and 8 µM ADP-ribose treatment in U-87MG cells, compared to the control group. It was confirmed that in the group using increasing radiation doses of 1, 2, and 5 Gy, the amount of ADP-ribose increased to a maximum at 4 hours, then began to decrease from 8 hours, and recovered to almost the same amount as that of the untreated group at 24 hours, but the group treated with ADP-ribose maintained a significant (P<0.001) increase in the amount of ADP-ribose in the cells compared to the control group even after 24 hours (FIG. 17A).

### Example 7-2: Change over time in ADP-ribose by treatment with various doses of radiation and ADP-ribose in Caki-1 cells

Caki-1 cells were divided into five groups: no treatment group (Control group), groups treated with 1, 2, and 5 Gy of radiation, and ADP-ribose treatment group, and cultured in a 6-well plate for 0, 4, 8, 16, and 24 hours, respectively. Then, the medium was removed, and the supernatant was prepared by treatment with RIPA buffer and 1% sodium dodecyl sulfate (SDS). The prepared supernatant was used for ELISA analysis for the detection of ADP-ribose.

FIG. 17B is a graph showing the intracellular increase (%) in ADP-ribose according to increasing radiation doses of 1, 2, and 5 Gy and 16 µM ADP-ribose treatment in Caki-1 cells, compared to the control group. It was confirmed that in the group using increasing radiation doses of 1, 2, and 5 Gy, the amount of ADP-ribose increased to a maximum at 4 hours and recovered to almost the same amount as that of the untreated group from 8 hours, but the group treated with ADP-ribose maintained a significant (P<0.001) increase in the amount of ADP-ribose in the cells compared to the control group even after 24 hours (FIG. 17B).

### Example 7-3: Change over time in ADP-ribose by treatment with various doses of radiation and ADP-ribose in AsPC-1 cells

AsPC-1 cells were divided into five groups: no treatment group (Control group), groups treated with 1, 2, and 5 Gy of radiation, and ADP-ribose treatment group, and cultured in a 6-well plate for 0, 4, 8, 16, and 24 hours, respectively. Then, the medium was removed, and the supernatant was prepared by treatment with RIPA buffer and 1% sodium dodecyl sulfate (SDS). The prepared supernatant was used for ELISA analysis for the detection of ADP-ribose.

FIG. 17C is a graph showing the intracellular increase (%) in ADP-ribose according to increasing radiation doses of 1, 2, and 5 Gy and 2 µM ADP-ribose treatment in AsPC-1 cells, compared to the control group. It was confirmed that in the group using increasing radiation doses of 1, 2, and 5 Gy, the amount of ADP-ribose increased to a maximum at 4 hours and recovered to almost the same amount as that of the untreated group from 16 hours, but the group treated with ADP-ribose maintained a significant (P<0.001) increase in the amount of ADP-ribose in the cells compared to the control group even after 24 hours (FIG. 17C).

### Example 7-4: Change over time in ADP-ribose by treatment with various doses of radiation and ADP-ribose in MDA-MB-231 cells

MDA-MB-231 cells were divided into five groups: no treatment group (Control group), groups treated with 1, 2, and 5 Gy of radiation, and ADP-ribose treatment group, and cultured in a 6-well plate for 0, 4, 8, 16, and 24 hours, respectively. Then, the medium was removed, and the supernatant was prepared by treatment with RIPA buffer and 1% sodium dodecyl sulfate (SDS). The prepared supernatant was used for ELISA analysis for the detection of ADP-ribose.

FIG. 17D is a graph showing the intracellular increase (%) in ADP-ribose according to increasing radiation doses of 1, 2, and 5 Gy and 16 µM ADP-ribose treatment in MDA-MB-231 cells, compared to the control group. It was confirmed that in the group using increasing radiation doses of 1, 2, and 5 Gy, the amount of ADP-ribose increased to a maximum at 4 hours and recovered to almost the same amount as that of the untreated group from 16 hours, but the group treated with ADP-ribose maintained a significant (P<0.001) increase in the amount of ADP-ribose in the cells compared to the control group even after 24 hours (FIG. 17D).

Summarizing the above, it was confirmed in Example 7 that external ADP-ribose treatment in various solid cancer cells could maintain the increase in intracellular ADP-ribose concentration above the concentration of ADP-ribose in cancer cells that elevated upon irradiation (FIG. 17). The anti-cancer effect of irradiation could be expected through the death action caused by damage to genetic material, but to overcome this, cancer cells continuously repair DNA strands through a process known as adenosine diphosphate ribosylation. However, as can be appreciated in FIG. 17, if ADP-ribose, which increases only temporarily for the repair of DNA strands destroyed by irradiation, is supported to accumulate continuously, disturbance of biochemical action may be caused, which may be expected to have a synergistic anti-cancer effect of radiation therapy, and furthermore, a possibility of using anti-cancer radiation at a reduced dose to overcome resistance to radiation therapy and reduce side effects.

### Example 8: Change in cancer cell viability by combination treatment of ADP-ribose and radiation

### Example 8-1: Change in viability in U-87MG cells by combination treatment of ADP-ribose and tolerable dose of radiation

3 × 10³ Brain cancer (U-87MG) cells were cultured in a 96-well plate for 24 hours under conditions of 37°C and 5% CO₂, treated with 8 µM of ADP-ribose and 2 or 5 Gy dose of radiation individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when U-87MG cells were treated with 2 Gy dose of radiation alone, about 88% of cancer cells showed resistance to survival compared to the untreated group (control), but when treated with ADP-ribose in combination, the survival rate of cancer cells decreased significantly (P<0.001) to about 12.3% even when 2 Gy dose of radiation was used (FIG. 18A).

### Example 8-2: Change in viability in H1975 cells by combination treatment of ADP-ribose and tolerable dose of radiation

3 × 10³ H1975 cells were cultured in a 96-well plate for 24 hours under conditions of 37°C and 5% CO₂, treated with 2 µM of ADP-ribose and 2 or 5 Gy dose of radiation individually or in combination, and cultured for 24 hours under conditions of 37°C and 5% CO₂ again. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when H1975 cells were treated with 2 Gy dose of radiation alone, about 75% of cancer cells showed resistance to survival compared to the untreated group (Control), but when treated with ADP-ribose in combination, the survival rate of cancer cells decreased significantly (P<0.001) to about 8% even when 2 Gy dose of radiation was used (FIG. 18B).

### Example 8-3: Change in viability in AsPC-1 cells by combination treatment of ADP-ribose and tolerable dose of radiation

3 × 10³ AsPC-1 cells were cultured in a 96-well plate for 24 hours under conditions of 37°C and 5% CO₂, treated with 2 µM of ADP-ribose and 2 or 5 Gy dose of radiation individually or in combination, and cultured for 24 hours under conditions of 37°C and 5% CO₂ again. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when AsPC-1 cells were treated with 2 Gy dose of radiation alone, about 87.3% of cancer cells showed resistance to survival compared to the untreated group (Control), but when treated with ADP-ribose in combination, the survival rate of cancer cells decreased significantly (P<0.001) to about 8% even when 2 Gy dose of radiation was used (FIG. 18C).

### Example 8-4: Change in viability in Hep G2 cells by combination treatment of ADP-ribose and tolerable dose of radiation

3 × 10³ Hep G2 cells were cultured in a 96-well plate for 24 hours under conditions of 37°C and 5% CO₂, treated with 4 µM of ADP-ribose and 2 or 5 Gy dose of radiation individually or in combination, and cultured for 24 hours under conditions of 37°C and 5% CO₂ again. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when Hep G2 cells were treated with 2 Gy dose of radiation alone, about 82.3% of cancer cells showed resistance to survival compared to the untreated group (Control), but when treated with ADP-ribose in combination, the survival rate of cancer cells decreased significantly (P<0.001) to about 7.3% even when 2 Gy dose of radiation was used (FIG. 18D).

### Example 8-5: Change in viability in MDA-MB-231 cells by combination treatment of ADP-ribose and tolerable dose of radiation

3 × 10³ MDA-MB-231 cells were cultured in a 96-well plate for 24 hours under conditions of 37°C and 5% CO₂, treated with 16 µM of ADP-ribose and 2 or 5 Gy dose of radiation individually or in combination, and cultured for 24 hours under conditions of 37°C and 5% CO₂ again. Then, 10 ul of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when MDA-MB-231 cells were treated with 2 Gy dose of radiation alone, about 90.6% of cancer cells showed resistance to survival compared to the untreated group (Control), but when treated with ADP-ribose in combination, the survival rate of cancer cells decreased significantly (P<0.001) to about 11.3% even when 2 Gy dose of radiation was used (FIG. 18E).

### Example 8-6: Change in viability in HCT116 cells by combination treatment of ADP-ribose and tolerable dose of radiation

3 × 10³ HCT116 cells were cultured in a 96-well plate for 24 hours under conditions of 37°C and 5% CO₂, treated with 16 µM of ADP-ribose and 2 or 5 Gy dose of radiation individually or in combination, and cultured for 24 hours under conditions of 37°C and 5% CO₂ again. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when HCT116 cells were treated with 2 Gy dose of radiation alone, about 90.3% of cancer cells showed resistance to survival compared to the untreated group (Control), but when treated with ADP-ribose in combination, the survival rate of cancer cells decreased significantly (P<0.001) to about 14% even when 2 Gy dose of radiation was used (FIG. 18F).

### Example 8-7: Change in viability in Caki-1 cells by combination treatment of ADP-ribose and tolerable dose of radiation

3 × 10³ Caki-1 cells were cultured in a 96-well plate for 24 hours under conditions of 37°C and 5% CO₂, treated with 16 µM of ADP-ribose and 2 or 5 Gy dose of radiation individually or in combination, and cultured for 24 hours under conditions of 37°C and 5% CO₂ again. Then, 10 µl of 3-(4,5-dimethylthiazol-2-yl)-2,5-diphenyltetrazolium bromide reagent was added to each well and reacted for 1 hour. After the reaction was completed, the reagent was removed, 200 µl of dimethyl sulfoxide was added to each well, and the absorbance was measured to confirm cell viability.

As a result, it was confirmed that when Caki-1 cells were treated with 2 Gy dose of radiation alone, about 94.7% of cancer cells showed resistance to survival compared to the untreated group (Control), but when treated with ADP-ribose in combination, the survival rate of cancer cells decreased significantly (P<0.001) to about 22.7% even when 2 Gy dose of radiation was used (FIG. 18G).

Summarizing the above, it could be appreciated from Example 8 that when administered in combination with ADP-ribose and radiation, a synergistic anti-cancer effect on solid cancer was shown, and ADP-ribose could be employed not only as an anti-cancer drug for solid cancer but also as an anti-cancer adjuvant for radiation therapy (FIG. 18).

### Example 9: Change in tumor volume in animal models by combination treatment of ADP-ribose and tolerable dose of radiation

### Example 9-1: Change in tumor volume in animal models of brain cancer by the combination treatment of ADP-ribose and tolerable dose of radiation

Brain cancer (U-87MG) cells (1 × 10⁷) were inoculated into 5-week-old BALB/c nude mice on the back flank, and the mice were divided into three groups: the control group, 2 Gy dose of radiation alone, and combination administration of 2 Gy dose radiation + 10 mg/kg of ADP-ribose. When the tumors were grown to a volume of approximately 150 mm³, 2 Gy dose of radiation was irradiated twice at 1 Gy each, and 10 mg/kg of ADP-ribose was injected subcutaneously 3 times a week. The tumor size was measured using a digital caliper, and the results of changes in tumor volume were compared by the group.

As a result of measuring and comparing the volume of brain cancer over time, FIG. 19A showed that the final volume of tumors in the control group after the last dose was about 2545.2 mm³, and the final tumor volume of the group irradiated with 2 Gy dose of radiation alone was about 1472.1 mm3, which was about 42% lower than that of the control group, whereas the final tumor volume of the group after combination treatment of 2 Gy dose of radiation and ADP-ribose was about 725.2 mm3, which showed a significant reduction of about 71.5% (P<0.001) compared to the control group, and even compared to the group treated with 2 Gy dose of radiation alone, the tumor volume of the combination treatment group was significantly reduced by about 50.7% (P<0.001) (FIG. 19A).

### Example 9-2: Change in tumor volume in animal models of pancreatic cancer by combination administration of ADP-ribose and tolerable dose of radiation

Pancreatic cancer (AsPC-1) cells (1 × 10⁷) were inoculated into 5-week-old BALB/c nude mice on the back flank, and the mice were divided into three groups: the control group (Control), 2 Gy dose of radiation alone, and combination administration of 2 Gy dose radiation + 10 mg/kg of ADP-ribose. When the tumors were grown to a volume of approximately 150 mm³, 2 Gy dose of radiation was irradiated twice at 1 Gy each, and 10 mg/kg of ADP-ribose was injected subcutaneously 3 times a week. The tumor size was measured using a digital caliper, and the results of changes in tumor volume were compared by the group.

As a result of measuring and comparing the volume of pancreatic cancer over time, FIG. 19B showed that the final volume of tumors in the control group (Control) after the last dose was about 2384.3 mm³, and the final tumor volume of the group irradiated with 2 Gy dose of radiation alone was about 1725.5 mm3, which was about 27.6% lower than that of the control group, whereas the final tumor volume of the group after combination treatment of 2 Gy dose of radiation and ADP-ribose was about 736.2 mm³, which showed a significant reduction of about 69.1% (P<0.001) compared to the control group, and even compared to the group treated with 2 Gy dose of radiation alone, the tumor volume of the combination treatment group was significantly reduced by about 57.3% (P<0.001) (FIG. 19B).

### Example 9-3: Change in tumor volume in animal models of breast cancer by combination administration of ADP-ribose and tolerable dose of radiation

Breast cancer (MDA-MB-231) cells (1 × 10⁷) were inoculated into 5-week-old BALB/c nude mice on the back flank, and the mice were divided into three groups: the control group (Control), 2 Gy dose of radiation alone, and combination administration of 2 Gy dose radiation + 20 mg/kg of ADP-ribose. When the tumors were grown to a volume of approximately 150 mm3, 2 Gy dose of radiation was irradiated twice at 1 Gy each, and 20 mg/kg of ADP-ribose was injected subcutaneously 3 times a week. The tumor size was measured using a digital caliper, and the results of changes in tumor volume were compared by the group.

As a result of measuring and comparing the volume of breast cancer over time, FIG. 19C showed that the final volume of tumors in the control group (Control) after the last dose was about 2336.1 mm3, and the final tumor volume of the group irradiated with 2 Gy dose of radiation alone was about 1459.6 mm3, which was about 37.5% lower than that of the control group, whereas the final tumor volume of the group after combination treatment of 2 Gy dose of radiation and ADP-ribose was about 640.4 mm3, which showed a significant reduction of about 72.6% (P<0.001) compared to the control group, and even compared to the group treated with 2 Gy dose of radiation alone, the tumor volume of the combination treatment group was significantly reduced by about 56.1% (P<0.001).

### Example 9-4: Change in tumor volume in animal models of kidney cancer treated with ADP-ribose and tolerable dose of radiation

Kidney cancer (Caki-1) cells (1 × 10⁷) were inoculated into 5-week-old BALB/c nude mice on the back flank, and the mice were divided into three groups: the control group (Control), 2 Gy dose of radiation alone, and combination administration of 2 Gy dose radiation + 20 mg/kg of ADP-ribose. When the tumors were grown to a volume of approximately 150 mm³, 2 Gy dose of radiation was irradiated twice at 1 Gy each, and 20 mg/kg of ADP-ribose was injected subcutaneously 3 times a week. The tumor size was measured using a digital caliper, and the results of changes in tumor volume were compared by the group.

As a result of measuring and comparing the volume of kidney cancer over time, FIG. 19D showed that the final volume of tumors in the control group (Control) after the last dose was about 2620.5 mm³, and the final tumor volume of the group irradiated with 2 Gy dose of radiation alone was about 2454.5 mm³, which was about 6.3% lower than that of the control group, whereas the final tumor volume of the group after combination treatment of 2 Gy dose of radiation and ADP-ribose was about 786.4 mm³, which showed a significant reduction of about 70% (P<0.001) compared to the control group, and even compared to the group treated with 2 Gy dose of radiation alone, the tumor volume of the combination treatment group was significantly reduced by about 68% (P<0.001) (FIG. 19D).

Summarizing the above, it could be appreciated from the animal experiment of Example 9, which proceeded after the cell experiment of Example 8, that when administered in combination with ADP-ribose and radiation, a synergistic anti-cancer effect on solid cancer was shown, and ADP-ribose could be employed not only as an anti-cancer drug for solid cancer but also as an anti-cancer adjuvant for radiation therapy (FIG. 19).

### Example 10: Confirmation of toxicity in normal cells when treated with ADP-ribose

In the case of an anti-cancer drug, it is important to exhibit toxicity specifically to cancer cells without exhibiting toxicity to normal cells. Therefore, whether ADP-ribose exhibited toxicity to normal cells according to the present disclosure was evaluated.

As normal cells, human colon fibroblast (CCD-18Co) and human dermal papilla cell (HDPC) cells were used. First, the respective normal cells (5 × 10³) were cultured in DMEM medium in a 96-well plate for 24 hours under conditions of 37°C and 5% CO₂. Then, the cells were divided into a total of 4 groups: ADP-ribose untreated group (Untreated), and treated groups (treated with increased concentrations of 25, 50, 100 ul). Cell viability was compared by MTT assay after 24, 48, and 72 hours of drug treatment.

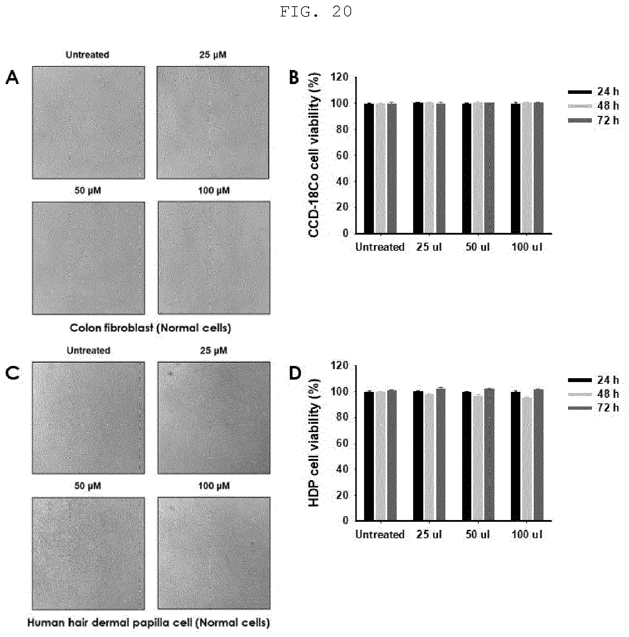

As a result, it was confirmed that no toxicity was observed when ADP-ribose was treated in all normal cells (FIGS. 20A and 20B: CCD-18Co, and FIGS. 20C and 20D: HDPC). Therefore, together with the experimental results of the Examples above, the present Example shows that ADP-ribose is capable of acting specifically on cancer cells to be usefully employed as an anti-cancer drug.

## Claims

1. An anti-cancer composition comprising: adenosine diphosphate ribose (ADP-ribose) or a pharmaceutically acceptable salt thereof.

2. The anti-cancer composition of claim 1, wherein the cancer is at least one solid cancer selected from the group consisting of brain cancer, lung cancer, pancreatic cancer, liver cancer, breast cancer, colon cancer, kidney cancer, stomach cancer, and ovarian cancer.

3. The anti-cancer composition of claim 1, wherein the cancer is a metastatic cancer.

4. The anti-cancer composition of claim 1, wherein the composition causes disturbance of biochemical action in cancer cells by inducing accumulation of ADP-ribose in cancer cells.

5. An anti-cancer adjuvant for radiation therapy comprising: adenosine diphosphate ribose (ADP-ribose) or a pharmaceutically acceptable salt thereof.

6. The anti-cancer adjuvant of claim 5, which improves radiation sensitivity.

7. An anti-cancer composition comprising: (i) adenosine diphosphate ribose (ADP-ribose) or a pharmaceutically acceptable salt thereof and (ii) a second anti-cancer drug.

8. The anti-cancer composition of claim 7, wherein the second anti-cancer drug is at least one selected from the group consisting of a cytotoxic anti-cancer drug, a targeted anti-cancer drug, and an immuno-oncology drug.

9. The anti-cancer composition of claim 8, wherein the second anti-cancer drug is the cytotoxic anti-cancer drug.

10. The anti-cancer composition of claim 9, wherein the cytotoxic anti-cancer agent is an alkylating agent, and the ADP-ribose or a pharmaceutically acceptable salt thereof enhances sensitivity to the alkylating agent.

11. The anti-cancer composition of claim 8, wherein the second anti-cancer drug is the targeted anti-cancer drug.

12. The anti-cancer composition of claim 11, wherein the targeted anti-cancer drug targets at least one target selected from the group consisting of VEGF/VEGFR, EGFR, and HER2, and the ADP-ribose or a pharmaceutically acceptable salt thereof enhances sensitivity to the targeted anti-cancer drug.

13. The anti-cancer composition of claim 12, wherein the targeted anti-cancer drug is a VEGF/VEGFR inhibitor for the treatment of brain or liver cancer.

14. The anti-cancer composition of claim 12, wherein the targeted anti-cancer drug is an EGFR inhibitor for the treatment of lung cancer.

15. The anti-cancer composition of claim 12, wherein the targeted anti-cancer drug is a HER2 inhibitor for the treatment of breast cancer.

16. The anti-cancer composition of claim 8, wherein the second anti-cancer drug is the immuno-oncology drug.

17. An anti-cancer adjuvant for chemotherapy comprising: adenosine diphosphate ribose (ADP-ribose) or a pharmaceutically acceptable salt thereof.

18. The anti-cancer adjuvant of claim 17, wherein an anti-cancer drug therapy and the anti-cancer adjuvant are administered at the same time or at different times.
